# EUROPEAN PATENT SPECIFICATION

(11) **EP 4 529 908 B1**
(45) Date of publication and mention of the grant of the patent: **30.09.2026**
(21) Application number: 23823790.3
(22) Date of filing: 07.06.2023
(51) Int. Cl.: A61F 13/15, A61F 13/49, A61F 13/51, B29C 65/08, B29C 65/00, B32B 5/02, B32B 5/26, B32B 37/00, B32B 37/20

(54) **STRETCHABLE SHEET MANUFACTURING DEVICE, STRETCHABLE SHEET MANUFACTURING METHOD, AND WEARABLE ARTICLE**
VORRICHTUNG ZUR HERSTELLUNG EINER DEHNBAREN FOLIE, VERFAHREN ZUR HERSTELLUNG EINER DEHNBAREN FOLIE UND WEARABLE-ARTIKEL
DISPOSITIF DE FABRICATION DE FEUILLE ÉTIRABLE, PROCÉDÉ DE FABRICATION DE FEUILLE ÉTIRABLE ET ARTICLE POUVANT ÊTRE PORTÉ

(30) Priority: 16.06.2022 JP 2022097660
(43) Date of publication of application: 02.04.2025
(73) Proprietor: Zuiko Corporation, Ibaraki-shi, Osaka 567-0082 (JP)
(72) Inventor: FUKUHARA, Takeshi, Ibaraki-shi, Osaka 567-0082 (JP); TSUSHIMA, Tatsuki, Ibaraki-shi, Osaka 567-0082 (JP); KOSHIJIMA, Miwa, Ibaraki-shi, Osaka 567-0082 (JP)
(74) Representative: Hasegawa, Kan
(86) International application number: PCT/JP2023/021082
(87) International publication number: WO 2023/243498

(56) References cited:
- EP-A1- 3 563 817
- EP-A1- 3 677 231
- EP-A1- 3 960 439
- EP-B1- 3 056 176
- WO-A1-2008/041639
- WO-A1-2018/070150
- WO-A1-2018/122970
- WO-A1-2020/235495
- WO-A1-2022/118691
- JP-A- 2020 054 744
- US-A1- 2019 231 606

## Description

### TECHNICAL FIELD

The present invention relates to a stretchable sheet manufacturing device, a stretchable sheet manufacturing method, and a wearable article including a stretchable sheet manufactured using this manufacturing device and manufacturing method.

### BACKGROUND ART

A wearable article having a stretchable waist-encircling member to be worn on a body of a wearer is known, as is disclosed in JP 6 903 810 B1.

The manufacture of the waist-encircling member of JP 6 903 810 B1 involves forming a stretchable sheet continuous body in which a pair of sheets and an elastic member disposed between the pair of sheets are integrated. The pair of sheets and the elastic member are partially joined together in the stretchable sheet continuous body. In the manufacture of the waist-encircling member, a process of weakening the elastic member is then performed in which the elastic member is partially cut in the stretchable sheet continuous body for a purpose such as joining another member (a fastener, an absorber, or the like) to a predetermined position on the waist-encircling member.

JP 2020 054744 A discloses a manufacturing method of a stretchable sheet which has a pair of bonding parts located on both sides of a rubber thread, puts the shrunk rubber thread between the bonding part pair and regulates a position of the rubber thread in the horizontal direction comprising: a first pair of bonding parts located nearer on one side in the horizontal direction than a cut position of the rubber thread; and a second pair of bonding parts adjacent to the first pair of bonding parts more on one side in a horizontal direction. The rubber thread is cut in the state that a regulation part regulating shrinking movements of the rubber thread is provided for at least a part of the range from the cut position of the rubber thread to an end on the other side of the second pair of bonding parts in the horizontal direction.

EP 3 960 439 A1 discloses a composite stretchable member capable of improving design properties at the time of contraction, and a wearing article using the same. A composite stretchable member stretchable in a specific direction includes two sheets facing each other, and a plurality of elastic members extending along the specific direction and being stretchable in the specific direction between the sheets. The sheets are bonded to each other at a plurality of first bonding sections. Each first bonding section continuously extends along the intersecting direction intersecting the specific direction and intersects the plurality of elastic members. Each elastic member is bonded to each sheet at an intersection with the corresponding one of the first bonding sections. Each first bonding section has a wavy line shape extending in the intersecting direction with convex portions and concave portions that alternately appear continuously. Two adjacent first bonding sections are disposed with the convex portions or the concave portions that at least partially overlap each other.

EP 3 563 817 A1 discloses a method for manufacturing a sheet-like member having an elastic member attached to the sheet-like member. The sheet-like member is manufactured by inserting an elastic-member continuous body between a pair of mutually-opposing facing surfaces of a sheet-like member continuous body being continuous in the direction of transport, and then cutting the sheet-like member continuous body. The method includes: a fixing step of fixing to the facing surface with an adhesive the elastic-member continuous body being stretched; a forming step of forming joining portions that join the pair of facing surfaces to each other; and a cutting step of producing the sheet-like member and the elastic member by cutting the sheet-like member continuous body and the elastic-member continuous body. In the fixing step, a portion of the elastic-member continuous body that is to be an end portion of the elastic member is fixed to the at least one facing surface with use of the adhesive. In the forming step, the joining portions are formed on two sides of the elastic-member continuous body in a CD direction. In the cutting step, the elastic member that attempts to expand in the CD direction due to being cut is attached to the sheet-like member by being sandwiched and pressed in the CD direction between the joining portions on the two sides.

EP 3 677 231 A1 discloses an apparatus and method for manufacturing an elastic composite structure for an absorbent sanitary product includes a bonding unit configured to bond a first web layer to a second web layer via a bond pattern that includes at least one bond line having at least one pair of adjacent bonds. The bonding unit secures an elastic thread within a passage defined by the at least one pair of adjacent bonds. The passage has a cross-sectional area smaller than a cross-sectional area of the elastic thread in a non-tensioned state.

WO 2008/041639 A1 discloses a technique for improving stretchability etc. of a stretchable section of an absorbent article such as a paper diaper. In the stretchable section, an inner layer and an outer layer are fusion-bonded together at predetermined intervals near both ends in the lateral direction of the elastic stretchable member with tensile force applied to the elastic stretchable member. By this, the elastic stretchable member is fixed to the exterior sheet by friction force between the stretchable member and the exterior sheet.

US 2019/231606 A1 discloses an apparatus for manufacturing an elastic composite structure for an absorbent sanitary product includes an anvil with weld pattern comprising at least one anchoring region and at least one deactivating region. The anchoring region includes anchoring welds that form anchoring bonds that fuse facing web layers and anchor elastic thread(s) in position relative to the facing web layers. The deactivating region includes a break bar constructed to sever the thread(s). A method of manufacturing the elastic composite structure includes positioning a tensioned elastic thread between web layers, fusing the web layers to form an anchored zone that includes anchoring bonds that fuse the web layers and anchor the tensioned elastic thread therebetween, and cutting the thread to form a deactivated zone between adjacent portions of the anchored zone that is free of tensioned threads. The method further includes fusing the web layers within the deactivated zone.

EP 3 056 176 B1 discloses an elastic structure for absorbent sanitary products, comprising a sleeve comprising a first layer and a second layer overlapping each other, at least one elastic thread, and a plurality of first connecting portions spaced apart in a longitudinal direction, wherein each of said first connecting portions comprises two first welds arranged on opposite sides of the thread, wherein the first welds weld said first and second layers to each other in a first longitudinal portion of said sleeve, and a plurality of second connecting portions spaced apart in the longitudinal direction, wherein each of said second connecting portions comprises at least one second weld, wherein said second welds weld said first and second layers to each other in a second longitudinal portion of said sleeve without axially connecting the thread to said second longitudinal portion of the sleeve.

### SUMMARY OF THE INVENTION

### <Technical Problem>

In the abovementioned weakening process, the elastic member is cut while in a stretched state. Therefore, the elastic member contracts due to restoring force during the weakening process. In a case where the elastic member has a high elongation rate before being cut, the restoring force may cause the elastic member to contract forcefully, the joining between the sheets and the elastic member may come undone, and the weakening process may extend to areas that do not require weakening.

An object of the present invention is to provide a device and method for manufacturing a stretchable sheet in which a stretchable sheet continuous body having a pair of sheets and an elastic member disposed between the pair of sheets is formed, wherein even if a weakening process of cutting the elastic member is performed on the stretchable sheet continuous body, it is possible to suppress adverse events in which the joining of the sheets and the elastic member in the stretchable sheet continuous body from coming undone. Another object of the present invention is to provide a wearable article comprising a stretchable sheet manufactured using this stretchable sheet manufacturing device and manufacturing method.

### <Solution to Problem>

A stretchable sheet manufacturing device of the present invention comprises a former and a cutter. The former is configured to form a stretchable sheet continuous body in which a first sheet that is continuous, a second sheet that is continuous, and an elastic member that is continuous and is disposed between the first sheet and the second sheet are integrated. The cutter is configured to cut the elastic member of the stretchable sheet continuous body. The former has a first joining member and a second joining member. The first joining member is configured to support the first sheet, the elastic member, and the second sheet, which are conveyed in a conveyance direction. The second joining member is configured to sandwich, together with the first joining member, the first sheet, the elastic member, and the second sheet. The first joining member has a first protrusion and a second protrusion in a facing surface that faces the second joining member. The second protrusion is disposed adjacent to the first protrusion in the conveyance direction of the first sheet, the elastic member, and the second sheet conveyed while supported by the first joining member. The second joining member is configured to form a restricting part in the stretchable sheet continuous body by sandwiching, together with the first protrusion of the first joining member, the elastic member, the first sheet, and the second sheet to weld the first sheet and the second sheet together. The second joining member is configured to form a fixed part in the stretchable sheet continuous body by sandwiching, together with the second protrusion of the second joining member, the elastic member, the first sheet, and the second sheet to weld the elastic member and at least one of the first sheet and the second sheet together. The cutter is configured to cut the elastic member of the conveyed stretchable sheet continuous body in the restricting part of the stretchable sheet continuous body. In the restricting part of the stretchable sheet continuous body, the first sheet and the second sheet are not joined to the elastic member. In addition, in the restricting part of the stretchable sheet continuous body, contraction movement of the elastic member cut by the cutter is restricted by the first sheet and the second sheet.

A method of manufacturing a stretchable sheet using a stretchable sheet manufacturing device comprising a former and a cutter, according to the present invention, comprises a forming step and a cutting step. In the forming step for integrating, by the former, a first sheet that is continuous, a second sheet that is continuous, and an elastic member that is continuous and is disposed between the first sheet and the second sheet to form a stretchable sheet continuous body. In the cutting step, by the cutter, the elastic member of the stretchable sheet continuous body is cut. The forming step includes a conveying step, a first forming step, and a second forming step. In the conveying step, the first sheet, the second sheet, and the elastic member are conveyed in a conveyance direction in a state in which the elastic member has been disposed between the first sheet and the second sheet. In the first forming step, the elastic member, the first sheet, and the second sheet conveyed in the conveyance direction are sandwiched between a first joining member that supports the first sheet, the elastic member, and the second sheet, and a second joining member, to weld the elastic member and at least one of the first sheet and the second sheet together so that a fixed part is formed in the stretchable sheet continuous body. In the second forming step, the elastic member, the first sheet, and the second sheet are sandwiched between the first joining member and the second joining member to weld the first sheet and the second sheet together so that a restricting part is formed in the stretchable sheet continuous body. In the cutting step, the elastic member of the stretchable sheet continuous body is cut in the restricting part of the stretchable sheet continuous body. The restricting part of the stretchable sheet continuous body is formed in a position adjacent to the fixed part on the stretchable sheet continuous body. In the restricting part of the stretchable sheet continuous body, the first sheet and the second sheet are not welded to the elastic member. In the restricting part of the stretchable sheet continuous body, contraction movement of the elastic member cut in the cutting step is restricted by the first sheet and the second sheet.

A wearable article of the present invention comprises a stretchable sheet. The stretchable sheet includes at least a first sheet piece, a second sheet piece, and an elastic member. The elastic member extends in a first direction and is disposed between the first sheet piece and the second sheet piece. The stretchable sheet includes, in the first direction, a first portion and a second portion. The elastic member is not present in the first portion. In the second portion, the elastic member is welded to at least one of the first sheet piece and the second sheet piece. In the first portion, there is a portion where, in a cross-sectional view sectioned in a direction orthogonal to the first direction, a maximum gap of a space between the first sheet piece and the second sheet piece, which communicates with a space of a third portion where the elastic member is placed, is smaller than a diameter of the elastic member while tension is not being applied.

### < Effects of Invention>

According to the stretchable sheet manufacturing device of the present invention, it is possible to suppress the incidence of adverse events in which the joining of the sheets and the elastic member comes undone in the stretchable sheet in the weakening process of cutting the elastic member.

According to the stretchable sheet manufacturing device in another example of the present invention, it is possible to suppress the incidence of adverse events in which the elastic member is cut during the joining of the elastic member and at least one of the first sheet and the second sheet, restoring force causes the elastic member to be pulled back upstream, and the stretchable sheet manufacturing device needs to be stopped in order to reposition the elastic member.

According to the stretchable sheet manufacturing method of the present invention, it is possible to suppress the incidence of adverse events in which the joining of the sheets and the elastic member comes undone in the stretchable sheet in the weakening process of cutting the elastic member.

According to the stretchable sheet manufacturing method in another example of the present invention, it is possible to suppress the incidence of adverse events in which the elastic member is cut during the joining of the elastic member and at least one of the first sheet and the second sheet, restoring force causes the elastic member to be pulled back upstream, and the stretchable sheet manufacturing device needs to be stopped in order to reposition the elastic member.

According to the wearable article of the present invention, it is possible to suppress the incidence of adverse events in which the fixing of the elastic member and the sheet pieces comes undone in the second portion of the stretchable sheet.

### BRIEF DESCRIPTION OF THE DRAWINGS

FIG. 1 is a front view of a refastenable wearable article in a state of being worn, pertaining to one embodiment of a wearable article having a stretchable sheet according to one embodiment of the present invention.
FIG. 2 is a rear view of the state of wearing of the wearable article of FIG. 1.
FIG. 3 is a laid-out view of the wearable article of FIG. 1 in an expanded state.
FIG. 4 is an exploded perspective view of the wearable article of FIG. 1.
FIG. 5 is a schematic diagram of a wearable article manufacturing device, which manufactures the wearable article of FIG. 1 and includes a stretchable sheet manufacturing device according to one embodiment of the present invention.
FIG. 6 is a diagram schematically depicting a stretchable sheet continuous body forming device of the wearable article manufacturing device of FIG. 5.
FIG. 7 is a schematic depiction of the forming device of FIG. 6, in which part of an external peripheral surface of an anvil roll is laid out in planar form.
FIG. 8 includes a drawing of a second groove formation portion enclosed within circle *α* in FIG. 7, as seen along a radial direction of the anvil roll, and a cross-sectional view of the second groove formation portion along arrow X1-X1 in said drawing.
FIG. 9 includes a drawing of a first groove formation portion enclosed within circle *β* in FIG. 7, as seen along a circumferential direction of the anvil roll, and a cross-sectional view of the first groove formation portion along arrow X2-X2 in said drawing.
FIG. 10 includes a drawing of a first groove formation portion enclosed within ellipse *γ* in FIG. 7, as seen along the circumferential direction of the anvil roll, and a cross-sectional view of the first groove formation portion along arrow X3-X3 in said drawing.
FIG. 11 is a drawing schematically depicting a stretchable sheet continuous body formed by the stretchable sheet manufacturing device of FIG. 5.
FIG. 12 is a partial cross-sectional view of the area indicated by B3 in FIG. 11, sectioned in a direction orthogonal to a conveyance direction of the stretchable sheet continuous body, the conveyance direction being a waist-encircling direction when the continuous body is formed on a wearable article.
FIG. 13 is a partial cross-sectional view of the area indicated by B1 in FIG. 11, sectioned in a direction orthogonal to the conveyance direction of the stretchable sheet continuous body, the conveyance direction being the waist-encircling direction when the continuous body is formed on a wearable article.
FIG. 14 is a diagram schematically depicting a stretchable sheet continuous body forming device of a stretchable sheet manufacturing device according to Modification A.
FIG. 15 is a diagram schematically depicting a stretchable sheet continuous body forming device of a stretchable sheet manufacturing device according to Modification C.

### DESCRIPTION OF EMBODIMENTS

Below is a description, made with reference to the drawings, of an embodiment of a stretchable sheet manufacturing device, a stretchable sheet manufacturing method, and a wearable article comprising a stretchable sheet, according to the present invention.

The embodiment described below is merely one example of the present invention and does not limit the scope of the present invention. It should be understood that a person skilled in the art could make various changes to the following embodiment without departing from the spirit and scope of the present invention as set forth in the claims.

### (1) Summary of wearable article

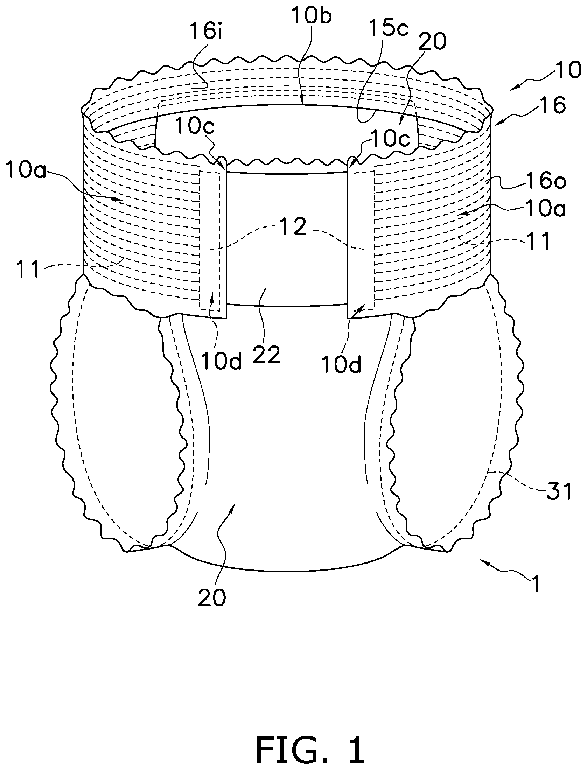

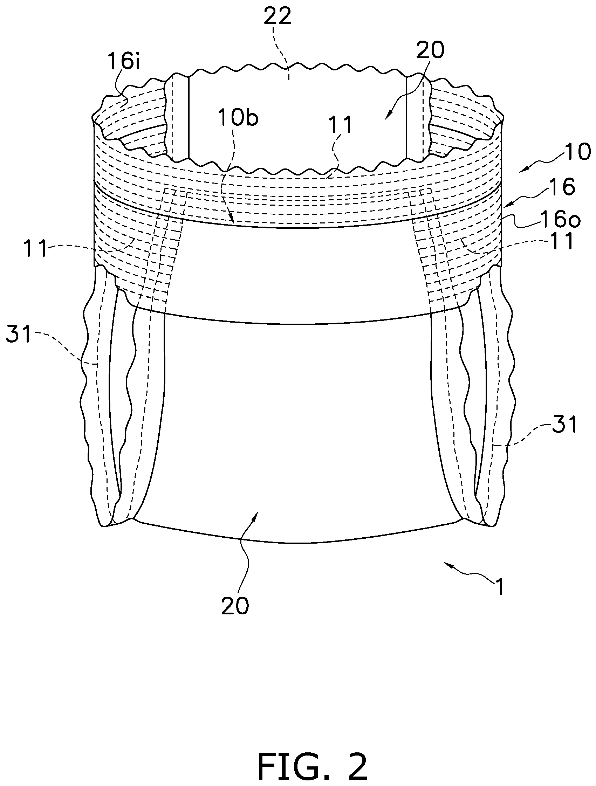

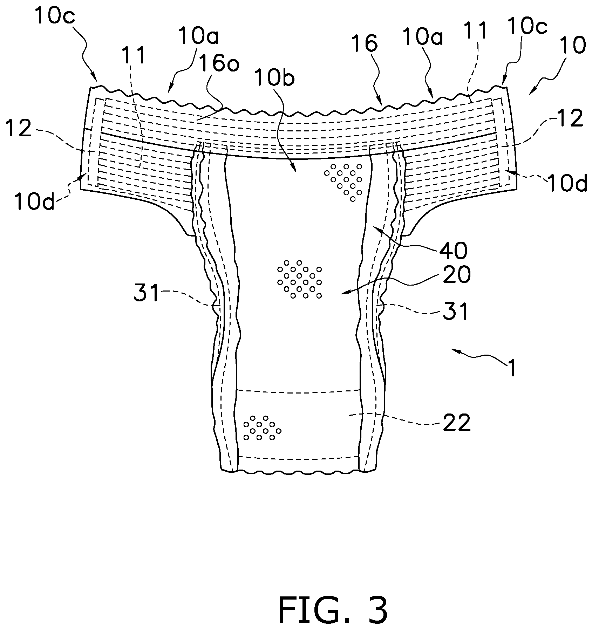

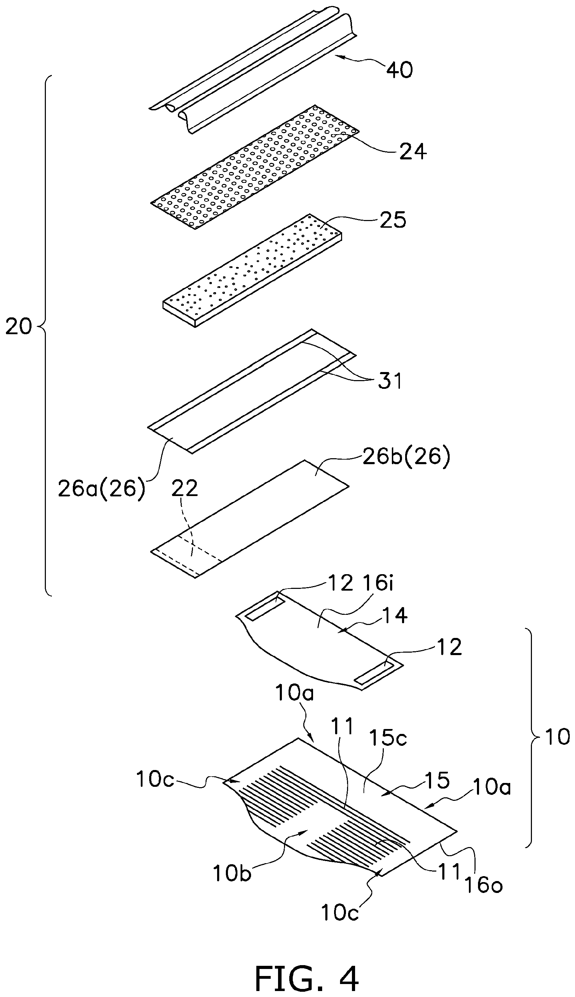

A summary of a wearable article to which a stretchable sheet according to the present invention is applied shall now be described with reference to FIGS. 1 to 4. FIG. 1 is a front view of a state of wearing of a refastenable wearable article 1. FIG. 2 is a rear view of the state of wearing of the wearable article 1. FIG. 3 is a laid-out view of the wearable article 1 in an expanded state. FIG. 4 is an exploded perspective view of the wearable article 1.

The wearable article 1 is a wearable article to which a stretchable sheet manufactured by a stretchable sheet manufacturing device 100 and a stretchable sheet manufacturing method according to one embodiment of the present invention is applied, the wearable article comprising an elastic waist-encircling member 10 worn on the body of a wearer. A stretchable sheet having an elastic member disposed between a pair of sheets is used as the waist-encircling member 10. The wearable article 1 is, in particular, a wearable article comprising a waist-encircling member 10 containing a stretchable sheet in which a weakening process has been performed on part of an elastic member (the elastic member has been partially cut).

The stretchable sheet of the present invention can be widely applied to, for example, waist-encircling members, sleeve opening members, hem opening members, etc., in disposable diapers, disposable medical gowns, etc. The purpose of use of the stretchable sheet are not limited.

In the present embodiment, a wearable article 1, which is a refastenable diaper, is used as an example to describe a wearable article comprising a stretchable sheet of the present invention. In this section, a summary of the structure and configuration of the wearable article 1 shall be briefly described, and the characteristic structure and configuration of the stretchable sheet of the present invention shall be described hereinafter. The wearable article 1 of the present embodiment has a structure and configuration similar to those of the wearable article disclosed in Patent Literature 1 (Japanese Patent Publication No. 6903810) in many respects. Therefore, a detailed description of the wearable article 1 shall be omitted unless otherwise necessary.

In the following description, the terms "waist-encircling direction" and "waist circumference orthogonal direction" may be used to explain directions and positions. "Waist-encircling direction" implies a direction along the waist of a wearer when the wearable article 1 is worn by the wearer. The waist circumference orthogonal direction means a direction orthogonal to the waist-encircling direction and along a body length direction (height direction) of the wearer when the wearable article 1 is worn by the wearer.

The refastenable wearable article 1 according to the present embodiment mainly comprises a waist-encircling member 10 disposed around the waist of the wearer, and an absorber 20 disposed from the back area of the wearer through the inseam area to the front abdomen, as shown in FIGS. 1 to 4.

The waist-encircling member 10 has a waist-encircling body 16 disposed around the waist of the wearer, and a male fastener 12 attached to the waist-encircling body 16, as shown in FIG. 3.

The waist-encircling body 16 is a member that is worn around the waist of the wearer and is capable of stretching in the waist-encircling direction. The stretchable sheet according to the present invention is used in the waist-encircling body 16. The waist-encircling body 16 mainly includes an inner sheet 14, an outer sheet 15, and an elastic member 11 disposed between the inner sheet 14 and the outer sheet 15, as shown in FIG. 4. One of the inner sheet 14 and the outer sheet 15 is an example of a first sheet piece in the present invention, and the other of the inner sheet 14 and the outer sheet 15 is an example of a second sheet piece in the present invention.

The inner sheet 14 is a sheet piece of, for example, nonwoven fabric. The inner sheet 14 is a band-form member extending in the waist-encircling direction. The inner sheet 14 has an inner surface 16i of the waist-encircling body 16 (see FIG. 4), which faces the skin of the wearer when the wearable article 1 is worn by the wearer. One end of the absorber 20 is joined to a portion of the inner sheet 14 that corresponds to an absorber j oining part 10b (a portion of the inner surface 16i of the waist-encircling body 16 that corresponds to the absorber joining part 10b) which will be described later. The male fastener 12 is joined to a male fastener joining part 10d (which will be described later) of the inner sheet 14 (a portion of the inner surface 16i of the waist-encircling body 16 that corresponds to the male fastener joining part 10d).

The outer sheet 15 is a sheet piece of, for example, nonwoven fabric. The outer sheet 15 is a band-form member extending in the waist-encircling direction. The outer sheet 15 has an outer surface 16o of the waist-encircling body 16 (see FIG. 4), which is disposed on the side opposite from the inner surface 16i of the waist-encircling body 16 and which faces outward (toward the side opposite of the skin of the wearer) when the wearable article 1 is worn by the wearer.

The elastic member 11 is a member that is disposed between the inner sheet 14 and the outer sheet 15 and that has elasticity so as to stretch in the waist-encircling direction, as in FIG. 4. In the manufactured wearable article 1, the elastic member 11 is in a stretched state, and restoring force acts on the elastic member 11 in a direction of contraction in the waist-encircling direction. Due to the waist-encircling body 16 having the elastic member 11, the waist-encircling body 16 can be caused to fit tightly to the waist of the wearer by the restoring force of the elastic member 11 when the wearer wears the wearable article 1. The elastic member 11 is formed of, for example, polyurethane, natural rubber, thermoplastic resin, or another material. Though not limited, the shape of the elastic member 11 is thread-form in the present embodiment. In the present embodiment, a plurality of thread-form elastic members 11 extending in the waist-encircling direction are disposed between the inner sheet 14 and the outer sheet 15 (see FIGS. 1 to 4).

The elastic member 11 is partially joined to at least one of the inner sheet 14 and the outer sheet 15. The elastic member 11 is a portion facing the inner sheet 14, and is disposed in an area of the waist-encircling body 16 excluding the absorber joining part 10b and end part 10c which will be described later, as shown in FIG. 4. In the absorber joining part 10b and end part 10c of the waist-encircling body 16 which will be described later, the elastic member 11 is cut by the weakening process in the process of manufacturing the wearable article 1, as shown in FIGS. 3 and 4.

The waist-encircling body 16, in which the inner sheet 14, the outer sheet 15, and the elastic member 11 are integrated, includes the absorber joining part 10b and two side parts 10a disposed on both sides of the absorber joining part 10b in the waist-encircling direction of the wearer, as shown in FIG. 3.

The absorber joining part 10b is a portion to which the absorber 20 is joined. The absorber joining part 10b is a center part of the waist-encircling body 16 in the waist-encircling direction, and is positioned in a lower part of the waist-encircling body 16 in the waist circumference orthogonal direction (at the side disposed at the bottom when the wearer is standing while wearing the wearable article 1).

In the absorber joining part 10b, since the process of weakening the elastic member 11 is performed as described above, the elastic member 11 is not present. Alternatively, as a result of the weakening process, the elastic force of the elastic member 11 does not substantially act on the absorber joining part 10b. The elastic member 11 is disposed at the upper part of the waist-encircling body 16 in the waist circumference orthogonal direction of the absorber joining part 10b (at the upper part of the absorber joining part 10b when the wearer is standing while wearing the wearable article 1). Therefore, in the upper part of the waist-encircling body 16 in the waist circumference orthogonal direction of the absorber joining part 10b, the waist-encircling body 16 can be caused to fit tightly to the waist of the wearer by the restoring force of the elastic member 11 when the wearer wears the wearable article 1.

Each of the two side parts 10a of the waist-encircling body 16 is a portion that extends in the waist-encircling direction from a position on the absorber joining part 10b to the end part 10c of the side part 10a in a direction away from the absorber joining part 10b. The two end parts 10c are the end parts of the waist-encircling body 16 in the waist-encircling direction. The side parts 10a are members that cover left and right side surfaces and a front surface of the waist of the wearer when the wearer wears the wearable article 1.

A male fastener 12 is joined to each of the two end parts 10c of the waist-encircling body 16. The male fasteners 12 work with a female fastener 22, which will be described later, in combination to function as a surface fastener. More specifically, the male fasteners 12 are joined to the inner surface 16i of the waist-encircling body 16 (the surface facing the wearer-skin-side of the inner sheet 14) at the end parts 10c of the waist-encircling body 16. The portions of the waist-encircling body 16 where the male fasteners 12 are joined are referred to as male fastener joining parts 10d.

The absorber 20 is a member for absorbing urine and other body fluids. The absorber 20 mainly has a core 25, a topsheet 24, an undersheet 26, the female fastener 22, leg gathers 31, and three-dimensional gathers 40.

The core 25 includes a nonwoven fabric carrying a highly absorbent polymer, and has the function of absorbing urine and other body fluids.

The topsheet 24 is a member provided on the wearer-skin-side of the core 25. The topsheet 24 is a sheet-form member made of one piece of nonwoven fabric, or a sheet-form member formed by layering multiple pieces of nonwoven fabric.

The undersheet 26 is a sheet-form member provided to the core 25 on the side opposite (outer surface side) from the wearer-skin-side so that the core 25 is sandwiched between the undersheet 26 and the topsheet 24. The undersheet 26 includes a liquid-impermeable sheet 26a, which is a nonwoven fabric made of polypropylene, polyethylene, or the like, and an exterior sheet 26b that covers the outside of the liquid-impermeable sheet 26a. The liquid-impermeable sheet 26a is a member that is water repellent and that prevents the passage of urine and other body fluids. The female fastener 22, which works with the male fasteners 12 of the waist-encircling body 16 in combination to function as a surface fastener, is attached to an outer surface of the undersheet 26 (the surface opposite from the wearer-skin-side of the exterior sheet 26b).

The leg gathers 31 are elastic members that are disposed at positions corresponding to the groin when the wearer wears the wearable article 1. The leg gathers 31 are provided along both edges of the liquid-impermeable sheet 26a in the waist-encircling direction and over substantially the entire liquid-impermeable sheet 26a in the waist circumference orthogonal direction when the wearable article 1 is laid out as shown in FIG. 3. The leg gathers 31 are disposed between the liquid-impermeable sheet 26a and the topsheet 24 in a stretched state in the waist circumference orthogonal direction when the wearable article 1 is laid out as shown in FIG. 3.

The three-dimensional gathers 40 are provided on a surface of the topsheet 24 facing the skin-surface side, as shown in FIG. 4. When the wearable article 1 is worn by a wearer, the three-dimensional gathers 40 elastically fit tightly to the back, crotch, front abdomen, etc., of the wearer, and suppress the outflow of urine and other body fluids to the outside of the wearable article 1.

The absorber 20 is joined to the absorber joining part 10b of the waist-encircling body 16, as shown in FIGS. 3 and 4. When the wearable article 1 is laid out (before being assembled in order to be worn), the absorber 20 extends in the waist circumference orthogonal direction from the absorber joining part 10b, in a direction away from the waist-encircling body 16, as shown in FIG. 3. When the wearer wears the wearable article 1, the absorber 20 is disposed from the back of the wearer through the inseam to the front abdomen. At the distal end part of the absorber 20 relative to the absorber joining part 10b in the waist circumference orthogonal direction, the aforementioned female fastener 22 is attached to the outer surface that faces the side opposite from the wearer when the wearer wears the wearable article 1.

In an assembled state (at the stage where the wearable article is used), the wearable article 1 is deformed such that the absorber 20 is folded back so that the topsheet 24 is disposed on the inner side, and the surfaces on the sides where the male fasteners 12 are disposed in at least the end parts 10c of the side parts 10a of the waist-encircling body 16 overlap the exterior sheet 26b of the undersheet 26 of the absorber 20. The male fasteners 12 of the waist-encircling body 16 are caused to engage the female fastener 22 attached to the exterior sheet 26b of the absorber 20, as shown in FIGS. 1 and 2. The wearer of the wearable article 1 pushes open the waist-encircling member 10 of the refastenable wearable article 1 assembled in underpants form with the male fasteners 12 and the female fastener 22 engaged, and inserts their legs to wear the wearable article 1 like underpants.

### (2) Summary of stretchable sheet manufacturing device and manufacturing method

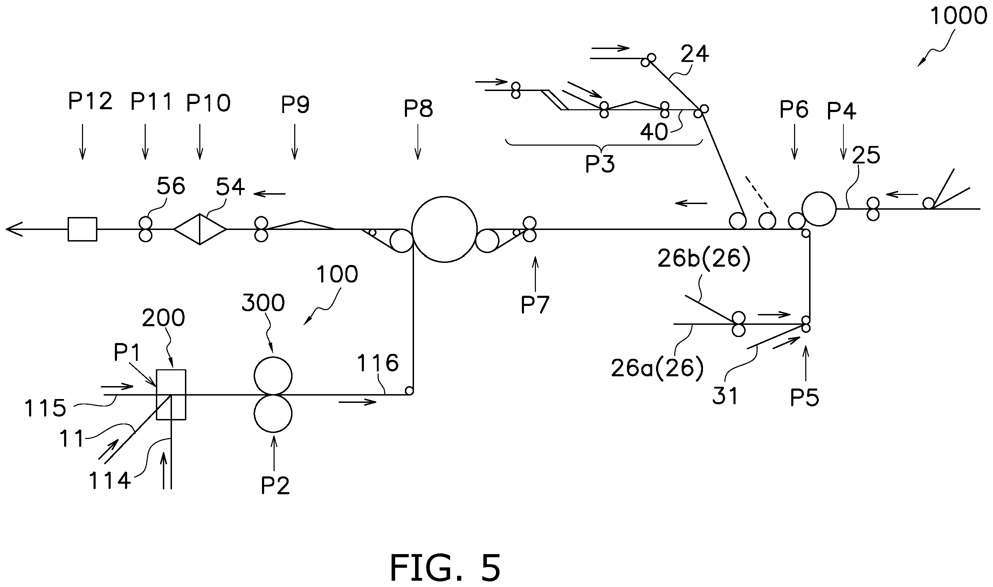

A device 1000 for manufacturing the wearable article 1, which includes a stretchable sheet manufacturing device 100, and a method for manufacturing the wearable article 1, which includes a step for manufacturing the stretchable sheet (the stretchable sheet continuous body), shall now be described with reference to FIG. 5.

The stretchable sheet manufacturing device 100, which constitutes part of the device 1000 for manufacturing the wearable article 1, is a device that forms a hereinafter-described stretchable sheet continuous body 116. The stretchable sheet manufacturing device 100 mainly includes a forming device 200 and cutting device 300 which will be described later. A characteristic configuration and method of the stretchable sheet manufacturing device 100 of the present embodiment shall be described hereinafter.

The device 1000 for manufacturing the wearable article 1 and the method for manufacturing the wearable article 1 are similar to the wearable article manufacturing device and manufacturing method disclosed in Patent Literature 1 (Japanese Patent Publication No. 6903810). Therefore, unless particularly necessary, detailed descriptions of the device 1000 for manufacturing the wearable article 1 and the method for manufacturing the wearable article 1 shall be omitted. In the following description, a direction in which sheets or the like are conveyed in each step is referred to as an MD direction, and a direction orthogonal to the MD direction is referred to as a CD direction.

Step P1 and step P2 in FIG. 5 are examples of steps for manufacturing the stretchable sheet continuous body 116, in which a plurality of waist-encircling bodies 16 are continuous.

### <Step P1>

In step P1, a continuous body 115 of a sheet in which a plurality of outer sheets 15 are continuous (an example of a second sheet) is conveyed in the MD direction, and on the continuous body 115, continuous elastic members 11 in a stretched state are disposed in a straight line along the MD direction, as shown in FIG. 5. A plurality of continuous elastic members 11 are disposed at intervals in the waist circumference orthogonal direction (CD direction) on the continuous body 115. Also in step P1, a continuous body 114 in which a plurality of inner sheets 14 are continuous (an example of a first sheet) is conveyed in the MD direction, and the continuous body 114 is disposed on the continuous body 115 so as to cover the elastic members 11 from above, as shown in FIG. 5.

Thus, when an elastic member 11 is disposed between the continuous body 115 and the continuous body 114 in a stretched state, the forming device 200, which is an example of a former, joins the elastic member 11 to at least one of the continuous body 115 and the continuous body 114. The forming device 200 joins the elastic member 11 to at least one of the continuous body 115 and the continuous body 114 by ultrasonic welding. The joining method used by the forming device 200 may be welding using heat sealing. A continuous body in which the continuous body 114, the elastic member 11, and the continuous body 115 are laminated and integrated by joining is hereinafter referred to as the stretchable sheet continuous body 116.

Details of the forming device 200 for joining the continuous body 115, the continuous body 114, and the elastic member 11 shall be described hereinafter.

### <Step P2>

In step P2, the weakening process is carried out on the elastic member 11 in positions of the stretchable sheet continuous body 116 corresponding to the absorber joining part 10b where the absorber 20 is disposed and the male fastener joining parts 10d where the male fasteners 12 are disposed. The weakening process is a process in which the elastic member 11 of the stretchable sheet continuous body 116 is cut by the cutting device 300. The cutting device 300 may be a gather cutter that cuts the elastic member 11 using a blade, or may be an embossing roll (heat embossing) device that applies heat to melt and cut the elastic member 11.

### <Steps P3 to P7>

Steps P3 to P7 are an example of steps for manufacturing the absorber 20.

In step P3, a partially processed topsheet is formed in which a continuous body of topsheets 24 in which a plurality of topsheets 24 are continuous and a continuous body of three-dimensional gathers 40 in which a plurality of three-dimensional gathers 40 are continuous are combined.

In step P4, a continuous body of cores 25 in which a plurality of cores 25 are continuous is formed, and a core 25 is manufactured using the continuous body of cores 25.

In step P5, a partially processed undersheet is formed in which a continuous body of undersheets 26 and a continuous body of leg gathers 31 are joined together

In step P6, a partially processed absorber is formed in which the partially processed topsheet formed in step P3, the core 25 formed in step P4, and the partially processed undersheet formed in step P5 are combined.

In step P7, the partially processed absorber formed in step P6 is cut, and individual absorbers 20 are manufactured.

### <Steps P8 to P11>

Steps P8 to P11 are an example of steps for assembling the wearable article 1.

In step P8, one end part (the end part on the side opposite from the female fastener 22) of the 90°-inverted absorber 20 manufactured in step P7 is disposed on the absorber joining parts 10b of the waist-encircling bodies 16 in the stretchable sheet continuous body 116 manufactured in step P2. The absorber 20 is joined to the inner surfaces 16i of the waist-encircling bodies 16 at positions corresponding to the absorber joining parts 10b. The joining means may be adhesion using a hot melt adhesive, thermal welding using a heat sealing device, or ultrasonic welding using an ultrasonic welding device.

In step P9, a portion (referred to as an extended part 15c, see FIG. 4) of the continuous body 115 of the outer sheets 15 of the stretchable sheet continuous body 116 that does not have the elastic member 11 disposed thereon and that is not covered by the continuous body 114 of the inner sheets 14 is folded back. The extended part 15c is joined to the absorbers 20 in a state of covering the end parts on the absorber joining parts 10b of the absorbers 20 (the end parts joined to the stretchable sheet continuous body 116). Also, in step P9, the male fasteners 12 are joined to portions of the waist-encircling bodies 16 that correspond to the male fastener joining parts 10d. The joining means in this embodiment may be adhesion using a hot melt adhesive, welding using heat sealing, or ultrasonic welding.

In step P10, the absorbers 20 are folded in two in the CD direction by two folding devices 54.

In step P11, the stretchable sheet continuous body 116 is cut in the CD direction by a cutter 56 at a position that is a midpoint between adjacent absorbers 20 and a midpoint between the male fasteners 12 in the MD direction. The stretchable sheet continuous body 116 and a plurality of absorbers 20 are thereby separated into individual waist-encircling bodies 16 and individual absorbers 20 joined thereto.

In step P12, the side parts 10a disposed on both sides of the waist-encircling member 10 in the MD direction are folded back toward the absorber joining part 10b of the waist-encircling member 10 so that the male fasteners 12 of both end parts 10c face the outer surface of the absorber 20. The male fasteners 12 of the waist-encircling member 10 and the female fastener 22 of the absorber 20 are then engaged with each other.

### (3) Forming device of stretchable sheet manufacturing device

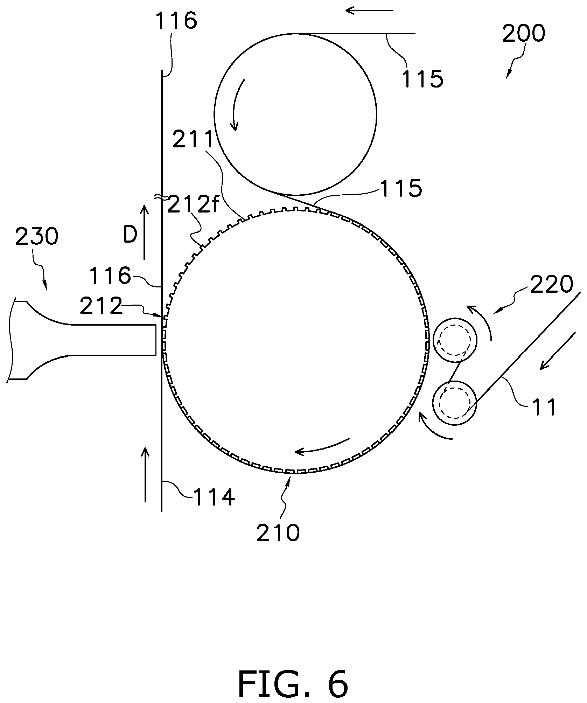

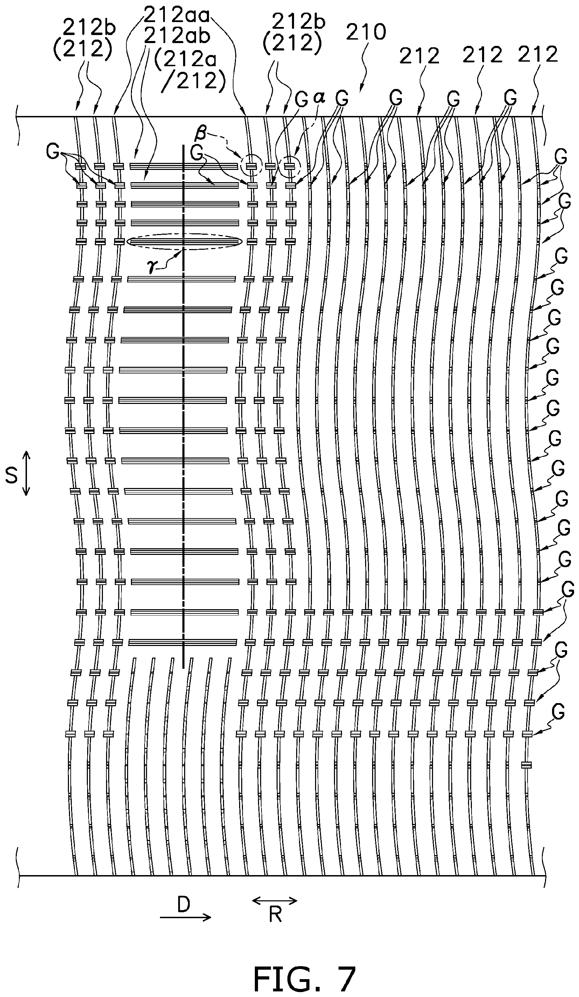

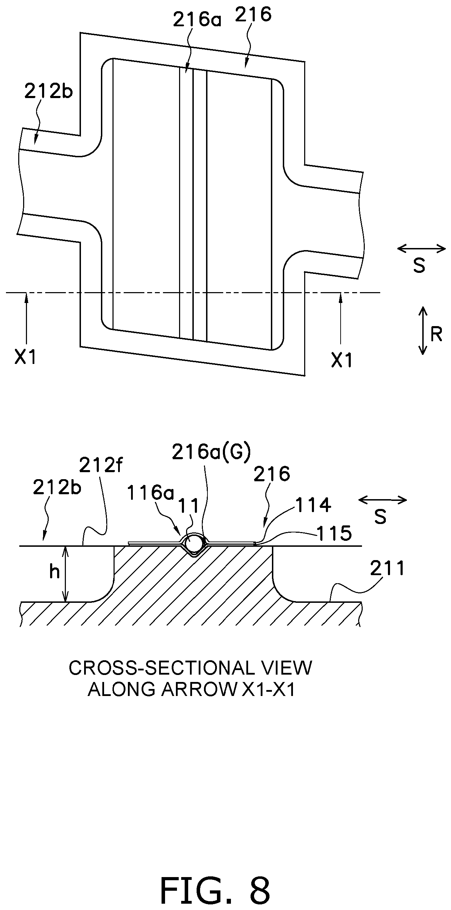

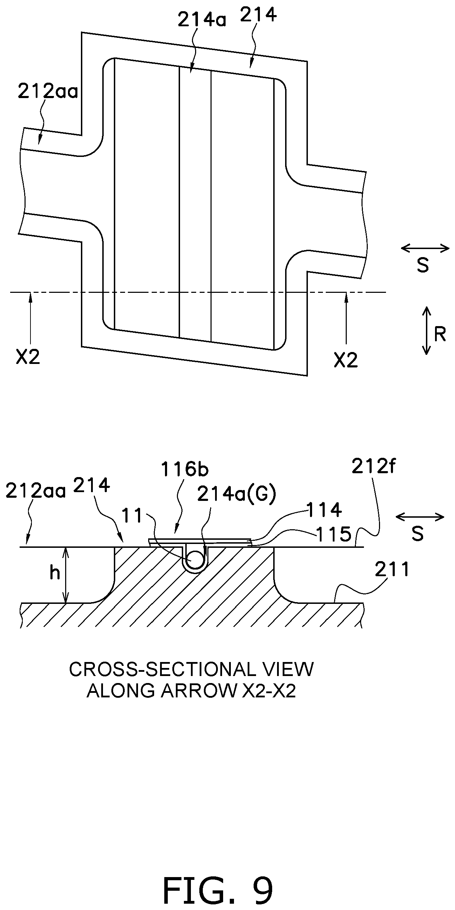

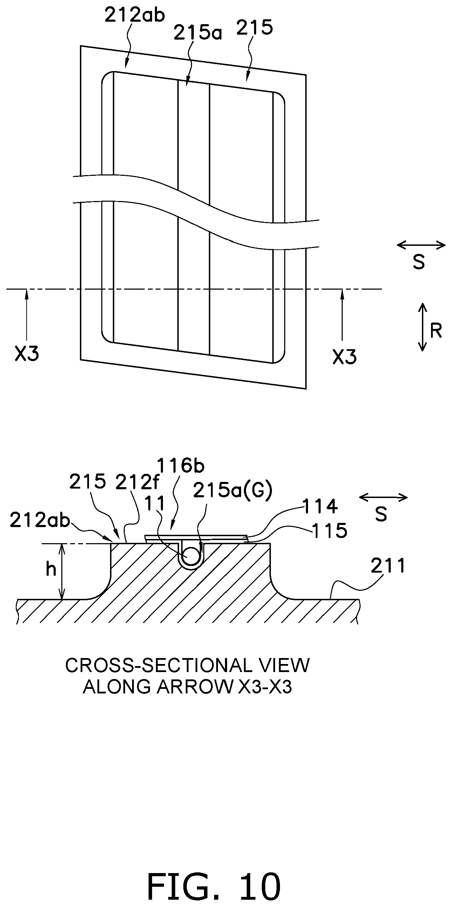

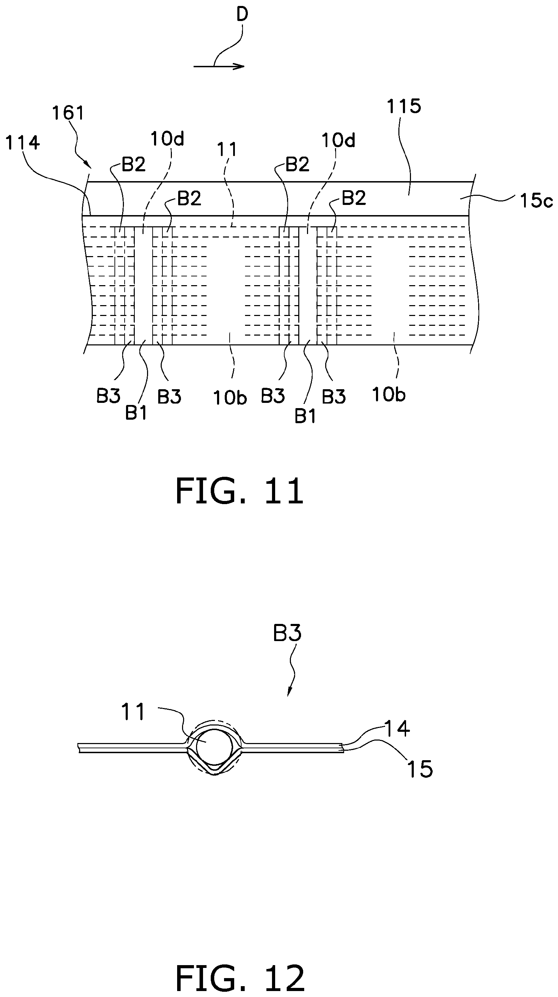

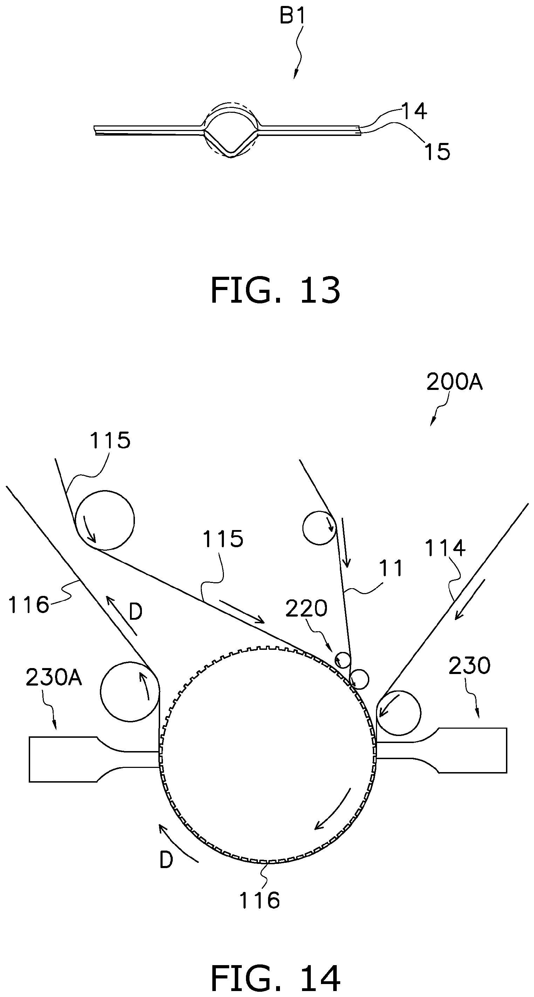

The forming device 200 of the stretchable sheet manufacturing device 100 shall now be described with reference to FIGS. 6 to 13. FIG. 6 is a diagram schematically depicting the forming device 200. FIG. 7 is a schematic depiction of the forming device 200, in which part of an external peripheral surface 211 of an anvil roll 210 is laid out in planar form. FIG. 8 includes a drawing of a second groove formation portion 216 (part of a second protrusion 212b) enclosed within circle *α* in FIG. 7, as seen along a radial direction of the anvil roll 210, and a cross-sectional view of the second groove formation portion 216 along arrow X1-X1 in said drawing. FIG. 9 includes a drawing of a first groove formation portion 214 (part of a first width-direction protrusion 212aa) enclosed within circle *β* in FIG. 7, as seen along a circumferential direction of the anvil roll 210, and a cross-sectional view of the first groove formation portion 214 along arrow X2-X2 in said drawing. FIG. 10 includes a drawing of a first groove formation portion 215 (a first circumferential-direction protrusion 212ab) enclosed within ellipse γ in FIG. 7, as seen along the radial direction of the anvil roll 210, and a cross-sectional view of the first groove formation portion 215 along arrow X3-X3 in said drawing. In a view of the first groove formation portion 215 of FIG. 10 along the radial direction of the anvil roll 210, the first groove formation portion 215 is depicted with a reduced length in a circumferential direction R of the anvil roll 210. FIG. 11 is a drawing schematically depicting a stretchable sheet continuous body 116 formed by the stretchable sheet manufacturing device 100 (the forming device 200 and the cutting device 300). FIG. 12 is a partial cross-sectional view of the area indicated by B3 (third area B3) in FIG. 11, sectioned in a direction orthogonal to a conveyance direction D (a waist-encircling direction when the continuous body is formed on a wearable article 1) of the stretchable sheet continuous body 11. FIG. 13 is a partial cross-sectional view of the area indicated by B1 (first area B1) in FIG. 11, sectioned in a direction orthogonal to the conveyance direction D (the waist-encircling direction when the continuous body is formed on a wearable article 1) of the stretchable sheet continuous body 11. FIGS. 6 to 13 are drawings for explanatory purposes, and do not accurately represent the actual dimensions and shapes of each component. In addition, in order to avoid the drawings becoming complicated, some reference numerals are omitted in FIGS. 6 to 13.

The forming device 200 is an example of a former, and forms the stretchable sheet continuous body 116 in which the continuous body 114 which is an example of a continuous first sheet, the continuous body 115 which is an example of a continuous second sheet, and the continuous elastic member 11 disposed between the continuous body 114 and the continuous body 115, are integrated.

The forming device 200 mainly has the anvil roll 210 which is an example of a first joining member, an introduction device 220, and an ultrasonic horn 230 which is an example of a second joining member (see FIG. 6). The ultrasonic horn 230 vibrates due to ultrasonic waves generated by an oscillator (not shown).

The anvil roll 210 is a member that supports the continuous body 114, the elastic member 11, and the continuous body 115, which are conveyed in the conveyance direction D (see the arrow in FIG. 6).

The introduction device 220 introduces the elastic member 11 into the anvil roll 210 while guiding the elastic member 11. In addition, the continuous body 115 is introduced into the anvil roll 210 upstream of the introduction device for the elastic member 11 in a rotation direction of the anvil roll 210. Furthermore, the continuous body 114 is introduced into the anvil roll 210 downstream of the introduction device for the elastic member 11 in the rotation direction of the anvil roll 210. The anvil roll 210 conveys the pair of continuous bodies 115, 114 and the elastic member 11 such that the elastic member 11 is disposed between the pair of continuous bodies 115, 114.

The ultrasonic horn 230, together with the anvil roll 210, sandwiches the continuous body 114, the elastic member 11, and the continuous body 115. The ultrasonic horn 230 operates in cooperation with the anvil roll 210 to partially join (ultrasonically weld) the pair of continuous bodies 115, 114. The ultrasonic horn 230 also operates in cooperation with the anvil roll 210 to partially join at least one of the pair of continuous bodies 115, 114 to the elastic member 11, causing the elastic member 11 to be held by the pair of continuous bodies 115, 114. The second joining member according to the present invention is not limited to the ultrasonic horn 230 and may be, *inter alia,* a roll having a built-in heater for thermally welding the continuous bodies 115, 114 to each other or the continuous bodies 115, 114 to the elastic member 11.

The anvil roll 210 shall now be described in greater detail.

The anvil roll 210 is a cylindrical member that rotates around an axis, and has numerous protrusions 212 that protrude radially outward from the anvil roll 210 on the external peripheral surface 211, as shown in FIGS. 6 and 7. The external peripheral surface 211 of the anvil roll 210 is an example of a facing surface of the anvil roll 210 serving as the first joining member, which faces the ultrasonic horn 230 serving as the second joining member. The protrusions 212 each have a first surface 212f that faces the ultrasonically vibrating ultrasonic horn 230 (see FIG. 6). The first surfaces 212f, for example, protrude outward in the radial direction of the anvil roll 210 by a certain height h from the external peripheral surface 211 of the anvil roll 210, as shown in FIGS. 8 to 10. The continuous body 115 and the continuous body 114 and sandwiched and pressurized between the first surfaces 212f of the protrusions 212 and the ultrasonically vibrating ultrasonic horn 230, whereby the continuous body 115 and the continuous body 114 are ultrasonically welded. Even though the continuous body 115 and the continuous body 114 are conveyed between the anvil roll 210 and the ultrasonic horn 230, the portions of these members that are not pressurized between the ultrasonic horn 230 and the first surfaces 212f of the protrusions 212 are not ultrasonically welded between the continuous body 115 and the continuous body 114.

The protrusions 212 are set apart from each other in the circumferential direction R of the anvil roll 210, as shown in FIG. 7. One or more grooves G extending in the circumferential direction R of the anvil roll 210 are formed on the first surfaces 212f of the protrusions 212. In other words, the grooves G extend along the conveyance direction D of the continuous body 114, the elastic member 11, and the continuous body 115 supported and conveyed by the anvil roll 210. The grooves G are grooves into which, among the continuous body 114, the elastic member 11, and the continuous body 115 supported and conveyed by the anvil roll 210, the continuous body 115 and the elastic member 11 are fitted. The grooves G provided on the first surfaces 212f of the protrusions 212 are disposed in a linear alignment with the grooves G provided on the adjacent protrusions 212 along the circumferential direction R of the anvil roll 210, as shown in FIG. 7. Therefore, the elastic member 11 introduced into the anvil roll 210 by the introduction device 220 are guided by the linearly aligned grooves G and conveyed along the circumferential direction R of the anvil roll 210.

The protrusions 212 include first protrusions 212a serving as an example of the first parts in the claims, and second protrusions 212b serving as an example of the second parts in the claims. The first protrusions 212a include first width-direction protrusions 212aa and first circumferential-direction protrusions 212ab.

The second protrusions 212b are provided in areas where first protrusions 212a are not provided in the circumferential direction R of the anvil roll 210. Specifically, in the circumferential direction R of the anvil roll 210, the second protrusions 212b are disposed on both sides of the areas where the first protrusions 212a are provided, adj acent to the areas where the first protrusions 212a are provided (see FIG. 7). In other words, in the conveyance direction D of the continuous body 114, the elastic member 11, and the continuous body 115 supported and conveyed by the anvil roll 210, the second protrusions 212b are disposed adjacent to upstream and downstream sides of the first protrusions 212a (see FIG. 7).

The second protrusions 212b, which are some of the protrusions 212, extend in a width direction S of the anvil roll 210 (the CD direction orthogonal to the MD direction in which the continuous bodies 114, 115 and the elastic member 11 are conveyed), as shown in FIG. 7. In other words, the second protrusions 212b extend along a generating line parallel to an axial direction of the anvil roll 210. Two second protrusions 212b are disposed adjacent to the upstream side and downstream side of each first protrusion 212a in the conveyance direction D (see FIG. 7). The number of second protrusions 212b is merely an example; one or three or more second protrusions 212b may be disposed adjacent to the upstream side and downstream side of each first protrusion 212a in the conveyance direction D. In each second protrusion 212b extending in the width direction S, there are formed a plurality of grooves G that extend in the circumferential direction R of the anvil roll 210 and that are separated from each other in the width direction S of the anvil roll 210, as shown in FIG. 7.

The first circumferential-direction protrusions 212ab, which are some of the protrusions 212, extend in the circumferential direction R of the anvil roll 210 (the MD direction in which the continuous bodies 114, 115 and the elastic member 11 are conveyed), as shown in FIG. 7. One groove G extending in the circumferential direction R of the anvil roll 210 is formed in each first circumferential-direction protrusion 212ab extending in the circumferential direction R. Each first circumferential-direction protrusion 212ab may be divided into a plurality in the circumferential direction R of the anvil roll 210 instead of extending lengthwise in the circumferential direction R of the anvil roll 210.

The first width-direction protrusions 212aa, which are some of the protrusions 212, extend in the width direction S of the anvil roll 210 (the CD direction orthogonal to the MD direction in which the continuous bodies 114, 115 and the elastic member 11 are conveyed), as shown in FIG. 7. In other words, the first width-direction protrusions 212aa extend along a generating line parallel to the axial direction of the anvil roll 210. One first width-direction protrusion 212aa is disposed adjacent to the upstream side and downstream side of each first circumferential-direction protrusion 212ab (between a first circumferential-direction protrusion 212ab and a second protrusion 212b) in the conveyance direction D (see FIG. 7). The number of the first width-direction protrusions 212aa is an example; two or more first width-direction protrusions 212aa may be disposed adjacent to the upstream side and downstream side of each first circumferential-direction protrusion 212ab in the conveyance direction D. In addition, the first width-direction protrusions 212aa may be omitted in another example. In each first width-direction protrusion 212aa extending in the width direction S, there are formed a plurality of grooves G that extend in the circumferential direction R of the anvil roll 210 and that are separated from each other in the width direction S of the anvil roll 210.

The grooves G provided in the first protrusions 212a and the second protrusions 212b are disposed in linear alignment with the grooves G provided in the first protrusions 212a or second protrusions 212b that are adjacent along the circumferential direction R of the anvil roll 210, as shown in FIG. 7.

The formation of the stretchable sheet continuous body 116 by the forming device 200 shall now be described.

In the forming device 200, when the continuous body 114, the elastic member 11, and the continuous body 115 are conveyed in the conveyance direction D, the continuous body 115 and the elastic member 11 fit into the grooves G of the plurality of protrusions 212 disposed along the circumferential direction R of the anvil roll 210, the grooves G being disposed in alignment in the circumferential direction R of the anvil roll 210 (disposed in the same position in the width direction S). In other words, the forming device 200 conveys the continuous body 114, the elastic member 11, and the continuous body 115 in the conveyance direction D in a state in which the continuous body 115 and the elastic member 11 are in the grooves G. In this example, it is assumed that the continuous body 115 and the elastic member 11 are in the grooves G, but this example is not provided by way of limitation; the continuous body 114 and the elastic member 11 may be in the grooves G (in other words, in the forming device 200, the continuous body 114, the elastic member 11, and the continuous body 115 may be conveyed with the continuous body 114 disposed on the inner side).

With the elastic member 11 sandwiched in between, the continuous body 114 and the continuous body 115 are conveyed along the external peripheral surface 211 of the anvil roll 210, and when the continuous body 114, the elastic member 11, and the continuous body 115 pass between the ultrasonic horn 230 and the protrusions 212, the ultrasonic horn 230 and the protrusions 212 of the anvil roll 210 sandwich the continuous body 114, the elastic member 11, and the continuous body 115 therebetween. The ultrasonic horn 230 also ultrasonically vibrates at this time.

As a result, the continuous body 114 and the continuous body 115, which are sandwiched between the ultrasonic horn 230 and the first surfaces 212f of the protrusions 212 of the anvil roll 210 and are pressurized between the ultrasonic horn 230 and the first surfaces 212f of the protrusions 212 of the anvil roll 210, are joined (ultrasonically welded) together.

In a case where the elastic member 11 is pressed by the ultrasonic horn 230 and the groove surfaces of the grooves G of the protrusions 212 of the anvil roll 210 via the continuous body 114 and the continuous body 115 at the time when the continuous body 114, the elastic member 11, and the continuous body 115 pass between the ultrasonic horn 230 and the protrusions 212 and the ultrasonic horn 230 is caused to ultrasonically vibrate, the continuous body 114 and the continuous body 115 are joined (ultrasonically welded) to the elastic member 11.

The protrusions 212 of the anvil roll 210 include the first protrusions 212a and the second protrusions 212b as described above. The continuous body 114 and the continuous body 115 are not joined to the elastic member 11 between the ultrasonic horn 230 and the first protrusions 212a, but at least one of the continuous body 114 and the continuous body 115 is joined to the elastic member 11 between the ultrasonic horn 230 and the second protrusions 212b. The details shall now be described.

The first protrusions 212a are parts that come into contact with portions that will become the male fastener joining parts 10d and with the outer sheet 15 surrounding these portions when the wearable article 1 is manufactured from the stretchable sheet continuous body 116 formed in the forming device 200. The first protrusions 212a, together with the ultrasonic horn 230, sandwich the continuous body 114, the elastic member 11, and the continuous body 115 and join the continuous body 114 and the continuous body 115 together, thereby forming restricting parts 116b in the stretchable sheet continuous body 116 (see FIGS. 9 and 10). More specifically, the first protrusions 212a form restricting parts 116b in the stretchable sheet continuous body 116 by sandwiching and pressurizing the continuous body 114 and the continuous body 115 between the first surfaces 212f and the ultrasonic horn 230 and joining the continuous body 114 and the continuous body 115 together.

In the restricting parts 116b, the continuous body 114 and the continuous body 115 are not joined to the elastic member 11. In addition, when the elastic member 11 is cut by the cutting device 300, the restricting parts 116b, by the continuous body 114 and the continuous body 115, restrict the contraction movement of the elastic member 11 cut by the cutting device 300. The restriction of the contraction movement of the elastic member 11 by the continuous body 114 and the continuous body 115 means not that the continuous body 114 and the continuous body 115 do not allow contraction movement of the elastic member 11 (the elastic member 11 does not contract), but rather that the continuous body 114 and the continuous body 115 restrict (suppress) momentum of the contraction movement of the elastic member 11. In other words, due to the presence of the restricting parts 116b, the elastic member 11 cut by the cutting device 300 contracts more slowly than if the restricting parts 116b were not present.

For restricting the contraction movement of the elastic member 11 with the restricting parts 116b, the elastic member 11 is in a state of being compressed by the continuous body 114 and the continuous body 115 in the restricting parts 116b, specifically. In other words, the restricting parts 116b of the stretchable sheet continuous body 116 have spaces to accommodate the elastic member 11 enclosed by the continuous body 114 and the continuous body 115, and in these spaces are portions where a maximum gap between the formed continuous body 114 and the continuous body 115 is smaller than a diameter of the elastic member 11 while tension is not being applied.

Next, the second protrusions 212b are parts that come into contact with the outer sheet 15 nearer to the absorber joining part 10b than the male fastener joining parts 10d in the waist-encircling direction when the wearable article 1 is manufactured from the stretchable sheet continuous body 116 formed in the forming device 200. The second protrusions 212b, together with the ultrasonic horn 230, sandwich the continuous body 114, the elastic member 11, and the continuous body 115 and join at least one of the continuous body 114 and the continuous body 115 to the elastic member 11 to form fixed parts 116a in the stretchable sheet continuous body 116. Both the continuous body 114 and the continuous body 115 are joined to the elastic member 11 at the fixed parts 116a in the present embodiment.

To form such restricting parts 116b and fixed parts 116a in the stretchable sheet continuous body 116, the first protrusions 212a and the second protrusions 212b preferably have configurations such as the following.

The second protrusions 212b have a plurality of second groove formation portions 216 (see FIG. 8) extending farther in the circumferential direction R of the anvil roll 210 than other portions of the second protrusions 212b, such as is depicted in circle *α* in FIG. 7. The plurality of second groove formation portions 216 are spaced apart from each other in the width direction S of the anvil roll 210.

In the first surfaces 212f of the second groove formation portions 216 are formed second grooves 216a (grooves G) (see FIG. 8) into which, of the continuous body 114, the elastic member 11, and the continuous body 115 conveyed in the conveyance direction D, the continuous body 115 and the elastic member 11 enter. There are no shape limitations, but the second grooves 216a are V-shaped as in FIG. 8. A depth of the second grooves 216a is smaller than a sum (first sum value) of the diameter of the elastic member 11 and a thickness of the continuous body 115 conveyed while under tension in the forming device 200. As a result, the elastic member 11 conveyed on the second grooves 216a of the second groove formation portions 216 is in a state of protruding at least partially from the second grooves 216a outward in the radial direction of the anvil roll 210, as in the cross-sectional view in FIG. 8. Therefore, when the continuous body 114, the elastic member 11, and the continuous body 115 are sandwiched between the ultrasonic horn 230 and the second groove formation portions 216 of the second protrusions 212b, the elastic member 11 is pressurized between the ultrasonic horn 230 and the second groove formation portions 216 via the continuous body 114 and the continuous body 115, and the continuous body 114 and the continuous body 115 are joined to the elastic member 11.

The first width-direction protrusions 212aa have a plurality of first groove formation portions 214 extending farther in the circumferential direction R of the anvil roll 210 than other portions of the first width-direction protrusions 212aa, as is depicted in circle *β* in FIG. 7. The plurality of first groove formation portions 214 are spaced apart from each other in the width direction S of the anvil roll 210. The first width-direction protrusions 212aa have first groove formation portions 214 shaped as described below at least in positions corresponding to the first circumferential-direction protrusions 212ab in the width direction S of the anvil roll 210.

In the first groove formation portions 214 are formed first grooves 214a (grooves G) into which, of the continuous body 114, the elastic member 11, and the continuous body 115 conveyed in the conveyance direction D, the continuous body 115 and the elastic member 11 enter. There are no shape limitations, but the first grooves 214a are U-shaped as in FIG. 9.

A depth of the first grooves 214a is larger than the sum (first sum value) of the diameter of the elastic member 11 and the thickness of the continuous body 115 conveyed while under tension in the forming device 200. As a result, the elastic member 11 conveyed on the first grooves 214a of the first groove formation portions 214 is in a state of being entirely accommodated in the first grooves 214a. Therefore, when the continuous body 114, the elastic member 11, and the continuous body 115 are sandwiched between the ultrasonic horn 230 and the first groove formation portions 214 of the first width-direction protrusions 212aa, the elastic member 11 is not pressurized between the ultrasonic horn 230 and the bottom surfaces of the first grooves 214a of the first groove formation portions 214, and the continuous body 114 and the continuous body 115 are not joined to the elastic member 11. When the continuous body 114, the elastic member 11, and the continuous body 115 are sandwiched between the ultrasonic horn 230 and the first groove formation portions 214 of the first width-direction protrusions 212aa, only the continuous body 114 and the continuous body 115 are joined together between the ultrasonic horn 230 and the first surfaces 212f of the first width-direction protrusions 212aa.

Furthermore, the depth of the first grooves 214a is preferably smaller than a sum (second sum value) of the diameter of the elastic member 11 and the thickness of the continuous body 115 in a state in which tension is not applied. As a result, when the elastic member 11 is partially cut as described hereinafter and the elastic member 11 returns to the original state (a natural length while not under tension) due to restoring force, pressure is applied to the elastic member 11 by the continuous body 114 and the continuous body 115, and contraction movement of the elastic member 11 is restricted by friction force between the elastic member 11 and at least one of the continuous body 114 and the continuous body 115.

It is preferable that a width of the first grooves 214a (a dimension in the width direction S of the anvil roll 210) is greater than a sum (third sum value) of the diameter of the elastic member 11 being conveyed through the forming device 200 in the conveyance direction D and twice the thickness of the continuous body 115, and is smaller than a sum (fourth sum value) of the diameter of the elastic member 11 and twice the thickness of the continuous body 115 while tension is not applied.

As a result, when the elastic member 11 is partially cut as described below and the elastic member 11 acts as though to return to the original state (the natural length while not under tension) due to restoring force, the diameter of the elastic member 11 becomes larger than the diameter while stretching. Therefore, the elastic member 11 is subjected to pressure by the continuous body 114 and the continuous body 115, and the contraction movement of the elastic member 11 is restricted by the friction force between the elastic member 11 and at least one of the continuous body 114 and the continuous body 115.

The first circumferential-direction protrusions 212ab each have a first groove formation portion 215 (see FIG. 10) that protrudes outward in the radial direction of the anvil roll 210 to the same height h as the first width-direction protrusions 212aa, as depicted in ellipse *γ* in FIG. 7. The first groove formation portions 215 differ from the first groove formation portions 214 by extending farther in the circumferential direction R of the anvil roll 210 than the first groove formation portions 214, but a cross-sectional shape of a first groove 215a formed in each first groove formation portion 215 is similar to that of the first grooves 214a (see FIG. 10). In addition, the first grooves 215a and the first grooves 214a serve similar purposes. The details of the first groove formation portions 215 and the first grooves 215a shall not be described here.

At the restricting parts 116b formed by the first protrusions 212a (the first circumferential-direction protrusions 212ab and the first circumferential-direction protrusions 212ab), the cutting device 300 cuts the elastic member 11 of the stretchable sheet continuous body 116 in the CD direction, which is orthogonal to the MD direction of the stretchable sheet continuous body 116. This configuration is not provided by way of limitation, but in this embodiment, the cutting device 300 cuts the elastic member 11 of the stretchable sheet continuous body 116 at center portions of the restricting parts 116b formed by the first protrusions 212a (the first circumferential-direction protrusions 212ab and the first circumferential-direction protrusions 212ab) in the waist-encircling direction (in the MD direction of the stretchable sheet continuous body 116). Due to the elastic member 11 being cut at these positions, the elastic member 11 moves at a relatively slow speed toward the fixed parts 116a adjacent to the restricting parts 116b in a state in which the contraction movement of the elastic member 11 is restricted by the restricting parts 116b, thereby forming one of the male fastener joining parts 10d of the waist-encircling body 16 manufactured from the stretchable sheet continuous body 116 conveyed downstream of the cutting device in the conveyance direction D, and one of the male fastener joining parts 10d of the waist-encircling body 16 manufactured from the stretchable sheet continuous body 116 conveyed upstream of the cutting device in the conveyance direction D.

FIG. 11 depicts a stretchable sheet continuous body 116 in which the elastic member 11 is partially cut by the cutting device 300 and absorber joining parts 10b and male fastener joining parts 10d are formed.

As a result of the restricting parts 116b being provided, first areas B1 that will become the first portions of the claims and third areas B3 that will become the third portions of the claims are formed in and around the male fastener joining parts 10d of the stretchable sheet continuous body 116 when the stretchable sheet continuous body 116 is applied to the waist-encircling body 16 as a stretchable sheet. In addition, as a result of the fixed parts 116a being provided, second areas B2 that will become the second portions of the claims are formed around the stretchable sheet continuous body 116 when the stretchable sheet continuous body 116 is applied to the waist-encircling body 16 as a stretchable sheet.

The first areas B1 are portions where the elastic member 11 is not present as a result of the elastic member 11 contracting. In the second areas B2, which correspond to the fixed parts 116a of the stretchable sheet continuous body 116, the elastic member 11 is joined to at least one of the continuous body 114 and the continuous body 115 (both in this embodiment). The third areas B3 are portions disposed between the first areas B1 and the second areas B2.

Each first area B1 includes a portion where, in a cross-sectional view sectioned in a direction orthogonal to the waist-encircling direction (generally, a first direction in which the elastic member 11 extends) (i.e., a cross-sectional view sectioned in a direction orthogonal to the conveyance direction D of the stretchable sheet continuous body 116) as in FIG. 13, a maximum gap of a space between the inner sheet 14 and the outer sheet 15, which communicates with a space in the second area B2 where the elastic member 11 is placed, is smaller than the diameter of the elastic member 11 (depicted by the double-dash line in FIG. 13) while tension is not being applied (the natural length). Furthermore/alternatively, each third area B3 includes a portion where, in a cross-sectional view sectioned in the direction orthogonal to the waist-encircling direction as in FIG. 12, the maximum gap of the space between the inner sheet 14 and the outer sheet 15, which communicates with the space in the second area B2 where the elastic member 11 is placed, is smaller than the diameter of the elastic member 11 (depicted by the double-dash line in FIG. 12) while tension is not being applied (the natural length).

The advantageous effects obtained due to the presence of the first areas B1 or the third areas B3 shall now be described.

In the first areas B1 and/or the third areas B3, the maximum gap of the space between the inner sheet 14 and the outer sheet 15 (referred to here as an elastic member placement space), which communicates with the space in the second area B2 where the elastic member 11 is placed, is smaller than the diameter of the elastic member 11 while tension is not being applied (the natural length).

Therefore, in the steps of manufacturing the stretchable sheet, when the cutting device 300 cuts the elastic member 11 and the elastic member 11 contracts and is thereby becoming absent in the elastic member placement space where the elastic member 11 had originally been placed in the first area B1, the contraction speed of the elastic member 11 can be reduced by sliding friction force generated between the elastic member 11 and at least one of the inner sheet 14 (continuous body 114) and the outer sheet 15 (continuous body 115).

Similarly, when the cutting device 300 cuts the elastic member 11 and the elastic member 11 contracts and is thereby becoming absent in the elastic member placement space where the elastic member 11 had originally been placed in the third area B3, the contraction speed of the elastic member 11 can be reduced by sliding friction force generated between the elastic member 11 and at least one of the inner sheet 14 (continuous body 114) and the outer sheet 15 (continuous body 115).

### (4) Characteristics

(4-1) A stretchable sheet manufacturing device 100 of the present embodiment comprises a forming device 200 serving as an example of a former, and a cutting device 300 serving as an example of a cutter. The forming device 200 forms a stretchable sheet continuous body 116 in which a continuous body 114 that is continuous, a continuous body 115 that is continuous, and an elastic member 11 that is continuous and is disposed between the continuous body 114 and the continuous body 115 are integrated. The cutting device 300 cuts the elastic member 11 of the stretchable sheet continuous body 116. The forming device 200 has an anvil roll 210 serving as an example of a first joining member, and an ultrasonic horn 230 serving as an example of a second joining member. The anvil roll 210 supports the continuous body 114, the elastic member 11, and the continuous body 115, which are conveyed in a conveyance direction D. The ultrasonic horn 230, together with the anvil roll 210, sandwiches the continuous body 114, the elastic member 11, and the continuous body 115. The anvil roll 210 has a first protrusion 212a serving as an example of a first part and a second protrusion 212b serving as an example of a second part in an external peripheral surface 211 serving as an example of a facing surface that faces the ultrasonic horn 230 serving as an example of the second joining member. The second protrusion 212b is disposed adjacent to the first protrusion 212a in the conveyance direction D. The ultrasonic horn 230 forms a restricting part 116b in the stretchable sheet continuous body 116 by sandwiching the elastic member 11, the continuous body 114, and the stretchable sheet continuous body 116 between the ultrasonic horn 230 and the first protrusion 212a of the anvil roll 210 to join the continuous body 114 and the continuous body 115 together. The ultrasonic horn 230 forms a fixed part 116a in the stretchable sheet continuous body 116 by sandwiching the elastic member 11, the continuous body 114, and the continuous body 115 between the ultrasonic horn 230 and the second protrusion 212b of the anvil roll 210 to join at least one of the continuous body 114 and the continuous body 115 to the elastic member 11. The cutting device 300 cuts the elastic member 11 of the conveyed stretchable sheet continuous body 116 in the restricting part 116b of the stretchable sheet continuous body 116. In the restricting part 116b of the stretchable sheet continuous body 116, the continuous body 114 and the continuous body 115 are not joined to the elastic member 11. In addition, in the restricting part 116b of the stretchable sheet continuous body 116, contraction movement of the elastic member 11 cut by the cutting device 300 is restricted by the continuous body 114 and the continuous body 115.

In the device 100 for manufacturing a wearable article 1, during a weakening process of cutting the elastic member 11, the incidence of adverse events in which the joining of the sheets and the elastic member 11 in the stretchable sheet continuous body 116 comes undone can be suppressed.

(4-2)
In the device 100 for manufacturing a wearable article 1 of the preferred embodiment, the first protrusion 212a of the anvil roll 210 includes first groove formation portions 214, 215, and the second protrusion 212b of the anvil roll 210 includes a second groove formation portion 216. First grooves 214a, 215a, into which the elastic member 11 and the continuous body 115 conveyed in the conveyance direction D enter and which extend along the conveyance direction D, are formed in the first groove formation portions 214, 215. A second groove 216a, into which the elastic member 11 and the continuous body 115 conveyed in the conveyance direction D enter and which extends along the conveyance direction D, is formed in the second groove formation portion 216. A depth of the first grooves 214a, 215a is greater than a first sum value, which is a sum of a diameter of the elastic member 11 and a thickness of the continuous body 115 being conveyed through the forming device 200 in the conveyance direction D. A depth of the second groove 216a is less than the first sum value.

In this device 100 for manufacturing a wearable article 1, it is possible to suppress the incidence of events in which, in the restricting part 116b, the elastic member 11 is joined to the continuous body 114 or the continuous body 115 and an elastic force of the elastic member 11 remains in a portion of the stretchable sheet continuous body 116 where the weakening process is to be performed.

(4-3)
In the device 100 for manufacturing a wearable article 1 of the preferred embodiment, a depth of the first grooves 214a, 215a is less than a second sum value, which is a sum of the diameter of the elastic member 11 and the thickness of the continuous body 115 while tension is not being applied.

In this device 100 for manufacturing a wearable article 1, rapid contraction movement of the elastic member 11 is restricted by the continuous body 114 and the continuous body 115 when the elastic member 11 is cut, and it is possible to suppress the incidence of adverse events in which the joining of the elastic member 11 with the continuous body 114 and/or the continuous body 115 comes undone in the fixed part 116a.

(4-4)
In the device 100 for manufacturing a wearable article 1 of the preferred embodiment, a width of the first grooves 214a, 215a in a width direction S of the anvil roll 210 is greater than a third sum value, which is a sum of the diameter of the elastic member 11 and twice the thickness of the continuous body 115 being conveyed through the forming device 200 in the conveyance direction D. The width of the first grooves 214a, 215a in the width direction of the anvil roll 210 is less than a fourth sum value, which is a sum of the diameter of the elastic member 11 and twice the thickness of the continuous body 115 while tension is not being applied.

In this device 100 for manufacturing a wearable article 1, it is possible to suppress the incidence of events in which the first grooves 214a, 215a inhibit the conveying of the elastic member 11 and the continuous body 115. Moreover, in this device 100 for manufacturing a wearable article 1, rapid contraction movement of the elastic member 11 is restricted by the continuous body 114 and the continuous body 115 when the elastic member 11 is cut, and it is possible to suppress the incidence of adverse events in which the joining of the elastic member 11 with the continuous body 114 and/or the continuous body 115 comes undone in a fixed part 116a.

(4-5)
A method for manufacturing a wearable article 1 of the present embodiment comprises a forming step and a cutting step. In the forming step, a continuous body 114 that is continuous, a continuous body 115 that is continuous, and an elastic member 11 that is continuous and is disposed between the continuous body 114 and the continuous body 115 are integrated, and a stretchable sheet continuous body 116 is formed. In the cutting step, the elastic member 11 of the stretchable sheet continuous body 116 is cut. The forming step includes a conveying step, a first forming step, and a second forming step. In the conveying step, the continuous body 114, the continuous body 115, and the elastic member 11 are conveyed in a conveyance direction D in a state in which the elastic member 11 has been disposed between the continuous body 114 and the continuous body 115. In the first forming step, the elastic member 11, the continuous body 114, and the continuous body 115 conveyed in the conveyance direction D are sandwiched between an anvil roll 210, which is an example of a first joining member that supports the continuous body 114, the elastic member 11, and the continuous body 115, and an ultrasonic horn 230, which is an example of a second joining member, to join the elastic member 11 with at least one of the continuous body 114 and the continuous body 115, whereby a fixed part 116a is formed in the stretchable sheet continuous body 116. In the second forming step, the elastic member 11, the continuous body 114, and the continuous body 115 are sandwiched between the anvil roll 210 and the ultrasonic horn 230 to join the continuous body 114 and the continuous body 115 together, whereby a restricting part 116b is formed in the stretchable sheet continuous body 116. The restricting part 116b of the stretchable sheet continuous body 116 is formed in a position adjacent to the fixed part 116a on the stretchable sheet continuous body 116. In the cutting step, the elastic member 11 of the stretchable sheet continuous body 116 is cut in the restricting part 116b of the stretchable sheet continuous body 116. In the restricting part 116b of the stretchable sheet continuous body 116, the continuous body 114 and the continuous body 115 are not joined to the elastic member 11. In addition, in the restricting part 116b of the stretchable sheet continuous body 116, contraction movement of the elastic member 11 cut in the cutting step is restricted by the continuous body 114 and the continuous body 115.

In this method for manufacturing a wearable article 1, in a weakening process of cutting the elastic member 11, the incidence of adverse events in which the joining of the sheets and the elastic member 11 in the stretchable sheet continuous body 116 comes undone can be suppressed.

(4-6)
A wearable article 1 of the present embodiment is a wearable article comprising a stretchable sheet. In particular, in the wearable article 1, a stretchable sheet manufactured by the stretchable sheet manufacturing device 100 is applied to a waist-encircling member 10 disposed around a waist of a wearer. The stretchable sheet applied to the waist-encircling member 10 includes at least an inner sheet 14, and outer sheet 15, and an elastic member 11. The elastic member 11 extends in a first direction (a waist-encircling direction along the waist of the wearer in the present embodiment), and is disposed between the inner sheet 14 and the outer sheet 15. The stretchable sheet applied to the waist-encircling member 10 includes, in the first direction, a first portion (first area B1) and a second portion (second area B2). The elastic member 11 is not present in the first area B1. In the second area B2, the elastic member 11 is joined to at least one of the inner sheet 14 and the outer sheet 15. In the first area B1, there is a portion where, in a cross-sectional view sectioned in a direction orthogonal to the first direction, a maximum gap of a space between the inner sheet 14 and the outer sheet 15, which communicates with a space in a second area B2 where the elastic member 11 is placed, is smaller than the diameter of the elastic member 11 while tension is not being applied (the natural length).

Due to the wearable article 1 having the configuration described above, contraction movement of the elastic member 11 can be restricted by the inner sheet 14 and the outer sheet 15 in the first area B1. Therefore, in the wearable article 1 of the present invention, it is possible to suppress the incidence of adverse events in which the fixing of the elastic member 11 and sheet pieces comes undone in the second area B2 of the waist-encircling member.

(4-7)
In the wearable article 1 of the present embodiment, the stretchable sheet applied to the waist-encircling member 10 includes a third portion (third area B3) disposed between the first area B1 and the second area B2 in a first direction (a waist-encircling direction along the waist of the wearer in the present embodiment). In the third portion, there is a portion where, in a cross-sectional view sectioned in a direction orthogonal to the first direction, a maximum gap of a space between the inner sheet 14 and the outer sheet 15, which communicates with the space of the second area B2 where the elastic member 11 is placed, is smaller than the diameter of the elastic member 11 while tension is not being applied (the natural length).

Due to the wearable article 1 having the configuration described above, contraction movement of the elastic member 11 can be restricted by the inner sheet 14 and the outer sheet 15 in the third area B3. Therefore, in the wearable article 1 of the present invention, it is possible to suppress the incidence of adverse events in which the fixing of the elastic member 11 and sheet pieces comes undone in the second area B2 of the waist-encircling member.

### (5) Modifications

Modifications relating to the above embodiment shall now be described.

### (5-1) Modification A

A description shall now be given, with reference to FIG. 14, of a device 200A for forming a stretchable sheet continuous body 116 in a stretchable sheet manufacturing device 100 according to Modification A. FIG. 14 is a diagram schematically depicting the forming device 200A.

Since the forming device 200A has many similarities to the forming device 200 of the above embodiment, attributes where the forming device 200A is different from the forming device 200 shall mainly be described.

The main difference in configuration between the forming device 200A and the forming device 200 is that the forming device 200A has two or more second joining members disposed along a conveyance direction D. In particular, in the present embodiment, the forming device 200A has, in addition to an ultrasonic horn 230 (farthest upstream second joining member), an ultrasonic horn 230A (farthest downstream second joining member) disposed downstream of the ultrasonic horn 230 in the conveyance direction D. The ultrasonic horn 230 and the ultrasonic horn 230A both, together with an anvil roll 210 serving as a first joining member, sandwich a continuous body 114, a continuous body 115, and an elastic member 11 conveyed in the conveyance direction D to join the continuous body 114 and the continuous body 115 together and join the elastic member 11 to at least one of the continuous body 114 and the continuous body 115. The ultrasonic horn 230 and the ultrasonic horn 230A may be, for example, horns for ultrasonic welding as depicted in FIG. 14, or rolls having built-in heaters instead.

The ultrasonic horn 230A serving as an example of a farthest downstream second joining member is disposed downstream of the ultrasonic horn 230 serving as an example of the farthest upstream second joining member in the conveyance direction D of the continuous body 114, the elastic member 11, and the continuous body 115. In FIG. 14, a position where the ultrasonic horn 230A and the anvil roll 210 face each other is disposed 180° away from a position where the ultrasonic horn 230 and the anvil roll 210 face each other in a circumferential direction of the anvil roll 210. However, FIG. 14 is merely one example of the arrangement of the ultrasonic horn 230 and the ultrasonic horn 230A, and an angle between the position where the ultrasonic horn 230A and the anvil roll 210 face each other and the position where the ultrasonic horn 230 and the anvil roll 210 face each other may be less than 180° or greater than 180° in the circumferential direction of the anvil roll 210.

The main functional difference between the forming device 200A and the forming device 200 is that the forming device 200A joins the continuous body 114 and the continuous body 115 together and joins the elastic member 11 and at least one of the continuous body 114 and the continuous body 115 together in two stages; a temporary joining using the ultrasonic horn 230, and a permanent joining using the ultrasonic horn 230A.

The reasons for performing welding in two stages in this manner shall first be described.

When the elastic member 11 is joined to at least one of the continuous body 114 and the continuous body 115, heat causes the elastic member 11 to decrease in strength, and there is a possibility that the elastic member 11 will be cut. If the elastic member 11 is cut, the restoring force of the elastic member 11 will cause the elastic member 11 to return to an upstream side in the conveyance direction, and it may be necessary to stop operation of the manufacturing device 100 in order to reposition the elastic member 11 between the continuous body 114 and the continuous body 115.

Therefore, in this modification, first, the elastic member 11 is temporarily joined between the continuous body 114 and the continuous body 115 under conditions in which the elastic member 11 is unlikely to break, and then the elastic member 11 is firmly joined (permanently) joined between the continuous body 114 and the continuous body 115 by welding. Due to this configuration, even if the elastic member 11 is cut during the permanent joining, it is possible to suppress the incidence of events in which the elastic member 11 moves upstream in the conveyance direction at the location where the elastic member 11 is temporarily joined between the continuous body 114 and the continuous body 115.

The term "temporarily joined" refers to a state in which the joined members are joined to each other in a separable manner. In other words, the term "temporarily joined" refers to a weak joined state in which materials are not broken up when the joined members are separated from each other. In addition, the term "permanently joined" refers to a state in which the joined members are joined to each other in an inseparable manner. In other words, the term "permanently joined" refers to a strong joined state in which, if the joined members were to be separated from each other, the material at least one of the members being separated would be broken up.

To realize such two-stage joining, seal strength between the ultrasonic horn 230A and the anvil roll 210 is set greater than seal strength between the ultrasonic horn 230 and the anvil roll 210.

For example, the ultrasonic horn 230 farthest upstream in the conveyance direction D, together with the anvil roll 210, sandwiches the continuous body 114, the elastic member 11, and the continuous body 115 at a comparatively weak welding pressure (first welding pressure), and, by ultrasonic welding, forms a temporary joined portion in which the elastic member 11 is temporarily joined to at least one of the continuous body 114 and the continuous body 115. The ultrasonic horn 230A farthest downstream in the conveyance direction D, together with the anvil roll 210, sandwiches the temporary joined portion at a comparatively strong welding pressure (second welding pressure greater than the first welding pressure), and by ultrasonic welding, permanently joins the elastic member 11 to at least one of the continuous body 114 and the continuous body 115. Although the numerical values are not limited, for example, the first welding pressure is 700 to 800 N, and the second welding pressure is 1250 N.

In cases such as when the ultrasonic horn 230 and the ultrasonic horn 230A are rolls having built-in heaters instead, the force with which the continuous body 114, the elastic member 11, and the continuous body 115 are sandwiched between the ultrasonic horns 230, 230A and the anvil roll 210 may be constant, and heater temperature may be set lower in the ultrasonic horn 230 and higher in the ultrasonic horn 230A.

With the forming device 200A, the stretchable sheet continuous body obtained from the permanent joining performed by the ultrasonic horn 230A may be the same as the stretchable sheet continuous body 116 described in the above embodiment.

However, the characteristics of the forming device 200A are also useful in the manufacture of a stretchable sheet continuous body that does not have the fixed parts 116a and the restricting parts 116b described in the above embodiment. In other words, the characteristics of the forming device 200A are useful even if the forming device 200A does not have a configuration including the first protrusions 212a and the second protrusions 212b.

A method for manufacturing a stretchable sheet in the forming device 200A shall now be described.

This stretchable sheet manufacturing method comprises a forming step in which a continuous body 114 serving as an example of a first sheet that is continuous, a continuous body 115 serving as an example of a second sheet that is continuous, and an elastic member 11 that is continuous and is disposed between the continuous body 114 and the continuous body 115 are integrated to form a stretchable sheet continuous body 116. The forming step includes a conveying step, a first forming step, and a second forming step.

In the conveying step, the continuous body 114, the continuous body 115, and the elastic member 11 are conveyed in the conveyance direction D in a state in which the elastic member 11 has been disposed between the continuous body 114 and the continuous body 115.

In the first forming step, the elastic member 11, the continuous body 114, and the continuous body 115 conveyed in the conveyance direction D are sandwiched between an anvil roll 210 serving as an example of a first joining member that supports the continuous body 114, the elastic member 11, and the continuous body 115, and an ultrasonic horn 230 serving as an example of an upstream second joining member, and a temporary joined portion is formed in which the elastic member 11 is temporarily joined to at least one of the continuous body 114 and the continuous body 115.

In the second forming step, the temporary joined portion is sandwiched between the anvil roll 210 and an ultrasonic horn 230A serving as an example of a downstream second joining member to permanently join the elastic member 11 to at least one of the continuous body 114 and the continuous body 115 and form a stretchable sheet continuous body 116.

Lastly, the characteristics of the device 100 for manufacturing a wearable article 1, the device 100 comprising a forming device 200A, shall be described.

The device 100 for manufacturing a wearable article 1 comprises a forming device 200A serving as an example of a former that forms a stretchable sheet continuous body 116 in which a continuous body 114 serving as an example of a first sheet that is continuous, a continuous body 115 serving as an example of a second sheet that is continuous, and an elastic member 11 that is continuous and is disposed between the continuous body 114 and the continuous body 115, are integrated. The forming device 200A has an anvil roll 210 serving as an example of a first joining member, and ultrasonic horns 230, 230A serving as examples of second joining members. The anvil roll 210 supports the continuous body 114, the elastic member 11, and the continuous body 115, which are conveyed in a conveyance direction D. Two or more (two in the present embodiment) second joining members are disposed along the conveyance direction D. The ultrasonic horns 230, 230A of the second joining members, together with the anvil roll 210, sandwich the continuous body 114, the elastic member 11, and the continuous body 115. At least the ultrasonic horn 230, which is farthest upstream in the conveyance direction D, together with the anvil roll 210, sandwiches the continuous body 114, the elastic member 11, and the continuous body 115 to form a temporary joined portion in which the elastic member 11 and at least one of the continuous body 114 and the continuous body 115 are temporarily joined together. The ultrasonic horn 230A, together with the anvil roll 210, sandwiches the temporary joined portion to permanently join the elastic member 11 and at least one of the continuous body 114 and the continuous body 115 together and form a stretchable sheet continuous body 116.

With this manufacturing device 100, it is possible to suppress the incidence of adverse events in which the elastic member 11 is cut during the joining of the elastic member 11 and at least one of the continuous body 114 and the continuous body 115, restoring force causes the elastic member 11 to be pulled back upstream, and the device 100 for manufacturing a wearable article 1 needs to be stopped in order to reposition the elastic member 11.

In this modification, an example of a case of two second joining members was described, but three or more second joining members may be disposed along the conveyance direction D. In such instances, at least the farthest upstream second joining member is preferably used in the formation of the temporary joined portion, and at least the farthest downstream second joining member is preferably used in the permanent joining of the elastic member and at least one of the first sheet and the second sheet.

### (5-2) Modification B

In Modification A, the elastic member 11 and at least one of the continuous body 114 and the continuous body 115 are joined together in the temporary joined portion. However, the elastic member 11 and at least one of the continuous body 114 and the continuous body 115 need not be joined together in the temporary joined portion.

Specifically, in the temporary joined portion, the continuous body 114 and the continuous body 115 alone may be joined together by welding or the like, and the elastic member 11 need not be joined to the continuous body 114 or the continuous body 115. In such an embodiment, even if the elastic member 11 is cut during permanent joining, the diameter of the elastic member 11 increases when the elastic member 11 is cut back to the natural length thereof, and it is therefore possible to prevent the incidence of events in which the elastic member 11 is sandwiched at the joining location of the continuous body 114 and the continuous body 115 and the elastic member 11 contracts and moves upstream in the conveyance direction.

### (5-3) Modification C

In the forming device 200A of Modification A, the ultrasonic horn 230 and the ultrasonic horn 230A are disposed for one anvil roll 210 serving as a first joining member, but the configuration of the forming device is not limited to such a configuration. The forming device may have two or more first joining members disposed along the conveyance direction D.

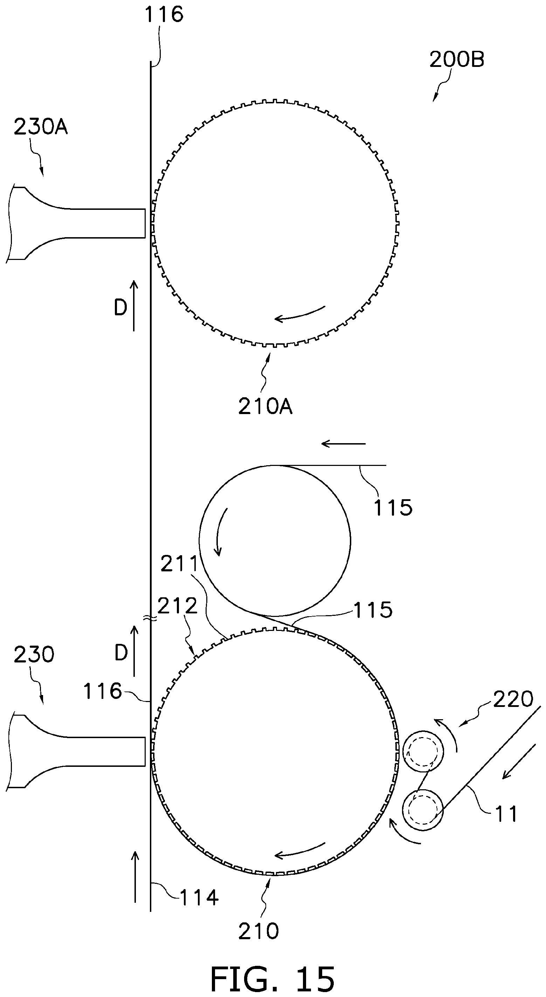

For example, as shown in FIG. 15, a forming device 200B may have an anvil roll 210A disposed farthest downstream in the conveyance direction D in addition to an anvil roll 210 disposed farthest upstream in the conveyance direction D. In the forming device 200B, the ultrasonic horn 230, together with the anvil roll 210, sandwiches the continuous body 114, the elastic member 11, and the continuous body 115 to form a temporary joined portion in which the elastic member 11 and at least one of the continuous body 114 and the continuous body 115 are temporarily joined together. In addition, in the forming device 200B, the ultrasonic horn 230A, together with the anvil roll 210A, sandwiches the temporary joined portion to permanently join the elastic member 11 and at least one of the continuous body 114 and the continuous body 115 together and form a stretchable sheet continuous body 116.

In such instances, it is preferable that the anvil roll 210 and the anvil roll 210A have protrusions 212 in the same pattern in the external peripheral surfaces 211, and the forming device 200B is designed such that the continuous body 115 comes into contact with the same (corresponding) positions on the anvil roll 210 and the anvil roll 210A. Specifically, it is preferable that the forming device 200B is designed such that if a portion of the continuous body 115 comes into contact with the first protrusions 212a of the anvil roll 210, that portion of the continuous body 115 will come into contact with the first protrusions 212a of the anvil roll 210A as well.

### (5-4) Modification D

In the above embodiment, an example was described in which the first joining member was an anvil roll, but the first joining member is not limited to a roll. For example, the first joining member may be a flat plate-form member in which protrusions 212 are provided in a surface facing the second joining member, such as is shown in FIG. 7.

### REFERENCE SIGNS LIST

- 1: Wearable article
- 10: Waist-encircling member (stretchable sheet)
- 11: Elastic member
- 14: Inner sheet (first sheet piece)
- 15: Outer sheet (second sheet piece)
- 100: Stretchable sheet manufacturing device
- 114: Continuous body (first sheet)
- 115: Continuous body (second sheet)
- 116: Stretchable sheet continuous body
- 116a: Fixed part
- 116b: Restricting part
- 200: Forming device (former)
- 210: Anvil roll (first joining member, farthest upstream first joining member)
- 210A: Anvil roll (first joining member, farthest downstream first joining member)
- 211: External peripheral surface (facing surface)
- 212a: First protrusion (first part)
- 212b: Second protrusion (second part)
- 214: First groove formation portion
- 214a: First groove
- 215: First groove formation portion
- 215a: First groove
- 216: Second groove formation portion
- 216a: Second groove
- 230: Ultrasonic horn (second joining member, upstream second joining member)
- 230A: Ultrasonic horn (second joining member, downstream second joining member)
- 300: Cutting device (cutter)
- B1: First area (first portion)
- B2: Second area (second portion)
- B3: Third area (third portion)
- D: Conveyance direction
- S: Width direction

## Claims

1. A stretchable sheet manufacturing device (100) comprising:
a former (200) configured to form a stretchable sheet continuous body (116) in which a first sheet (114) that is continuous, a second sheet (115) that is continuous, and an elastic member (11) that is continuous and is disposed between the first sheet and the second sheet are integrated; and
a cutter (300) configured to cut the elastic member of the stretchable sheet continuous body,
the former having a first joining member (210) configured to support the first sheet, the elastic member, and the second sheet, which are conveyed in a conveyance direction (D), and a second joining member (230) configured to sandwich, together with the first joining member, the first sheet, the elastic member, and the second sheet,
the first joining member having a first protrusion (212a) and a second protrusion (212b) disposed adjacent to the first protrusion in the conveyance direction in a facing surface (211) that faces the second joining member,
the second joining member is configured to:
form a restricting part (116b) in the stretchable sheet continuous body by sandwiching the elastic member, the first sheet, and the second sheet between the second joining member and the first protrusion of the first joining member and welding the first sheet and the second sheet together; and
form a fixed part (116a) in the stretchable sheet continuous body by sandwiching the elastic member, the first sheet, and the second sheet between the second joining member and the second protrusion of the first joining member and welding at least one of the first sheet and the second sheet to the elastic member,
the cutter is configured to cut the elastic member of the conveyed stretchable sheet continuous body in the restricting part of the stretchable sheet continuous body, and
in the restricting part, the first sheet and the second sheet being not welded to the elastic member and contraction movement of the elastic member cut by the cutter being restricted by the first sheet and the second sheet.

2. The stretchable sheet manufacturing device according to claim 1, wherein
the first protrusion (212a) includes a first groove formation portion (214, 215) in which a first groove (214a, 215a) into which the elastic member and the second sheet conveyed while under tension in the conveyance direction are configured to enter and extending along the conveyance direction is formed,
the second protrusion (212b) includes a second groove formation portion (216) in which a second groove (216a) into which the elastic member and the second sheet conveyed in the conveyance direction are configured to enter and extending along the conveyance direction is formed,
a depth of the first groove is greater than a first sum value, which is a sum of a diameter of the elastic member and a thickness of the second sheet being conveyed while under tension through the former in the conveyance direction, and
a depth of the second groove is less than the first sum value.

3. The stretchable sheet manufacturing device according to claim 2, wherein
the depth of the first groove (214a, 215a) is less than a second sum value, which is a sum of the diameter of the elastic member and the thickness of the second sheet while tension is not being applied.

4. The stretchable sheet manufacturing device according to claim 2 or 3, wherein
a width of the first groove (214a, 215a) in a width direction (S) of the first joining member (210) is
greater than a third sum value, which is a sum of the diameter of the elastic member and twice the thickness of the second sheet being conveyed while under tension through the former in the conveyance direction, and
less than a fourth sum value, which is a sum of the diameter of the elastic member and twice the thickness of the second sheet while tension is not being applied.

5. A method of manufacturing a stretchable sheet using a stretchable sheet manufacturing device (100) comprising a former (200) and a cutter (300), the method comprising:
a forming step (P1) for integrating, by the former, a first sheet (114) that is continuous, a second sheet (115) that is continuous, and an elastic member (11) that is continuous and is disposed between the first sheet and the second sheet to form a stretchable sheet continuous body; and
a cutting step (P2) for cutting, by the cutter, the elastic member of the stretchable sheet continuous body, wherein
the forming step includes:
a conveying step in which the first sheet, the second sheet, and the elastic member are conveyed in a conveyance direction (D) in a state in which the elastic member has been disposed between the first sheet and the second sheet;
a first forming step in which the elastic member, the first sheet, and the second sheet conveyed in the conveyance direction are sandwiched between a first joining member (210) that supports the first sheet, the elastic member, and the second sheet, and a second joining member (230) to weld the elastic member and at least one of the first sheet and the second sheet together so that a fixed part (116a) is formed in the stretchable sheet continuous body; and
a second forming step in which the elastic member, the first sheet, and the second sheet are sandwiched between the first joining member and the second joining member to weld the first sheet and the second sheet together so that a restricting part (116b) is formed in a position adjacent to the fixed part on the stretchable sheet continuous body,
in the cutting step, the elastic member of the stretchable sheet continuous body is cut in the restricting part of the stretchable sheet continuous body, and
in the restricting part, the first sheet and the second sheet are not welded to the elastic member, and contraction movement of the elastic member cut in the cutting step is restricted by the first sheet and the second sheet.

6. A wearable article (1) comprising a stretchable sheet (10), wherein:
the stretchable sheet includes at least a first sheet piece (14), a second sheet piece (15), and an elastic member (11) that extends in a first direction (D) and that is disposed between the first sheet piece and the second sheet piece;
the stretchable sheet includes, in the first direction,
a first portion (B1) where the elastic member is not present; and
a second portion (B2) where the elastic member is welded to at least one of the first sheet piece and the second sheet piece, and
in the first portion, there is a portion where, in a cross-sectional view sectioned in a direction orthogonal to the first direction, a maximum gap of a space between the first sheet piece and the second sheet piece, which communicates with a space of the second portion where the elastic member is placed, is smaller than a diameter of the elastic member while tension is not being applied.

7. The wearable article according to claim 6, wherein
the stretchable sheet further includes a third portion (B3) disposed between the first portion (B1) and the second portion (B2) in the first direction and where the elastic member is placed, and
in the third portion, there is a portion where, in a cross-sectional view sectioned in a direction orthogonal to the first direction, a maximum gap of a space between the first sheet piece and the second sheet piece, which communicates with the space of the second portion where the elastic member is placed, is smaller than the diameter of the elastic member while tension is not being applied.

## Patentansprüche

1. Vorrichtung (100) zur Herstellung einer dehnbaren Folie, umfassend:
eine Formvorrichtung (200), die dazu ausgelegt ist, einen Endloskörper (116) aus dehnbarer Folie zu bilden, in dem eine erste Endlosfolie (114), eine zweite Endlosfolie (115) und ein elastisches Element (11), das ebenfalls endlos ist und zwischen der ersten und der zweiten Folie angeordnet ist, integriert sind; und
eine Schneidevorrichtung (300), die dazu ausgelegt ist, das elastische Element des Endloskörpers der dehnbaren Folie zu schneiden,
wobei die Formvorrichtung ein erstes Verbindungselement (210), das dazu ausgelegt ist, die erste Bahn, das elastische Element und die zweite Bahn, die in einer Förderrichtung (D) befördert werden, zu stützen, sowie ein zweites Verbindungselement (230) aufweist, das dazu ausgelegt ist, zusammen mit dem ersten Verbindungselement die erste Bahn, das elastische Element und die zweite Bahn einzuklemmen,
wobei das erste Verbindungselement einen ersten Vorsprung (212a) und einen zweiten Vorsprung (212b) aufweist, der in Förderrichtung benachbart zum ersten Vorsprung in einer dem zweiten Verbindungselement zugewandten Fläche (211) angeordnet ist,
Das zweite Verbindungselement ist so ausgebildet, dass es:
einen Begrenzungsabschnitt (116b) in dem dehnbaren Folien-Endloskörper bildet, indem das elastische Element, die erste Folie und die zweite Folie zwischen dem zweiten Verbindungselement und dem ersten Vorsprung des ersten Verbindungselements eingeklemmt und die erste Folie und die zweite Folie miteinander verschweißt werden; und
einen festen Abschnitt (116a) in dem dehnbaren Folien-Endloskörper zu bilden, indem das elastische Element, die erste Folie und die zweite Folie zwischen dem zweiten Verbindungselement und dem zweiten Vorsprung des ersten Verbindungselements eingeklemmt und mindestens eine der ersten Folie und der zweiten Folie mit dem elastischen Element verschweißt werden,
wobei die Schneidevorrichtung so ausgebildet ist, dass sie das elastische Element des beförderten dehnbaren Folienendloskörpers im Begrenzungsabschnitt des dehnbaren Folienendloskörpers schneidet, und
wobei im Begrenzungsabschnitt die erste Folie und die zweite Folie nicht mit dem elastischen Element verschweißt sind und die Kontraktionsbewegung des durch die Schneidevorrichtung geschnittenen elastischen Elements durch die erste Folie und die zweite Folie begrenzt wird.

2. Vorrichtung zur Herstellung einer dehnbaren Bahn gemäß Anspruch 1, wobei
der erste Vorsprung (212a) einen ersten Nutbildungsabschnitt (214, 215) aufweist, in dem eine erste Nut (214a, 215a) ausgebildet ist, in die das elastische Element und die zweite Bahn, die unter Spannung in Förderrichtung befördert werden, eintreten können und die sich entlang der Förderrichtung erstreckt,
der zweite Vorsprung (212b) einen zweiten Nutbildungsabschnitt (216) aufweist, in dem eine zweite Nut (216a) ausgebildet ist, in die das elastische Element und die zweite Bahn, die in Förderrichtung befördert werden, eintreten können und die sich entlang der Förderrichtung erstreckt,
die Tiefe der ersten Nut ist größer als ein erster Summenwert, der sich aus der Summe des Durchmessers des elastischen Elements und der Dicke der zweiten Bahn ergibt, die unter Spannung in Förderrichtung durch die erste Nut befördert wird, und
die Tiefe der zweiten Nut ist kleiner als der erste Summenwert.

3. Vorrichtung zur Herstellung einer dehnbaren Folie nach Anspruch 2, wobei
die Tiefe der ersten Nut (214a, 215a) kleiner ist als ein zweiter Summenwert, der sich aus der Summe des Durchmessers des elastischen Elements und der Dicke der zweiten Folie im spannungsfreien Zustand ergibt.

4. Vorrichtung zur Herstellung einer dehnbaren Folie nach Anspruch 2 oder 3, wobei
die Breite der ersten Nut (214a, 215a) in einer Breitenrichtung (S) des ersten Verbindungselements (210)
größer ist als ein dritter Summenwert, der sich aus der Summe des Durchmessers des elastischen Elements und dem Doppelten der Dicke der zweiten Bahn ergibt, die unter Spannung in Förderrichtung durch die Form geführt wird, und
kleiner ist als ein vierter Summenwert, der sich aus der Summe des Durchmessers des elastischen Elements und dem Doppelten der Dicke der zweiten Bahn ergibt, wenn keine Spannung ausgeübt wird.

5. Verfahren zur Herstellung einer dehnbaren Folie unter Verwendung einer Vorrichtung (100) zur Herstellung dehnbarer Folien, die eine Formvorrichtung (200) und eine Schneidevorrichtung (300) umfasst, wobei das Verfahren umfasst:
einen Formungsschritt (P1), bei dem durch die Formvorrichtung eine erste, durchgehende Bahn (114), eine zweite, durchgehende Bahn (115) und ein elastisches Element (11), das durchgehend ist und zwischen der ersten und der zweiten Bahn angeordnet ist, zu einem durchgehenden Körper einer dehnbaren Bahn zusammengefügt werden; und
einen Schneideschritt (P2) zum Schneiden des elastischen Elements des dehnbaren, bahnförmigen Endloskörpers durch das Schneidwerkzeug, wobei
der Formungsschritt umfasst:
einen Förderschritt, bei dem die erste Bahn, die zweite Bahn und das elastische Element in einer Förderrichtung (D) in einem Zustand gefördert werden, in dem das elastische Element zwischen der ersten Bahn und der zweiten Bahn angeordnet wurde;
einen ersten Formungsschritt, bei dem das elastische Element, die erste Folie und die zweite Folie, die in der Förderrichtung befördert werden, zwischen einem ersten Verbindungselement (210), das die erste Folie, das elastische Element und die zweite Folie stützt, und einem zweiten Verbindungselement (230) eingeklemmt werden, um das elastische Element und mindestens eine der ersten Folie und der zweiten Folie miteinander zu verschweißen, so dass ein fester Abschnitt (116a) in dem dehnbaren Folien-Endloskörper gebildet wird; und
ein zweiter Formungsschritt, bei dem das elastische Element, die erste Folie und die zweite Folie zwischen dem ersten Verbindungselement und dem zweiten Verbindungselement eingeklemmt werden, um die erste Folie und die zweite Folie miteinander zu verschweißen, sodass ein Begrenzungsabschnitt (116b) an einer Stelle neben dem festen Abschnitt an dem dehnbaren Folien-Endloskörper gebildet wird,
im Schneideschritt wird das elastische Element des dehnbaren Folien-Endloskörpers im Begrenzungsabschnitt des dehnbaren Folien-Endloskörpers durchtrennt, und
im Begrenzungsabschnitt sind die erste Folie und die zweite Folie nicht mit dem elastischen Element verschweißt, und die Kontraktionsbewegung des im Schneideschritt durchtrennten elastischen Elements wird durch die erste Folie und die zweite Folie begrenzt.

6. Ein tragbarer Gegenstand (1), der eine dehnbare Folie (10) umfasst, wobei:
die dehnbare Folie mindestens ein erstes Folienstück (14), ein zweites Folienstück (15) und ein elastisches Element (11) umfasst, das sich in einer ersten Richtung (D) erstreckt und zwischen dem ersten Folienstück und dem zweiten Folienstück angeordnet ist;
die dehnbare Folie in der ersten Richtung
einen ersten Abschnitt (B1), in dem das elastische Element nicht vorhanden ist, und
einen zweiten Abschnitt (B2), in dem das elastische Element mit mindestens einem der ersten und zweiten Folienstücke verschweißt ist, umfasst, und
Im ersten Abschnitt gibt es einen Bereich, in dem in einer Querschnittsansicht, die in einer zur ersten Richtung orthogonalen Richtung geschnitten ist, der maximale Abstand zwischen dem ersten Blechteil und dem zweiten Blechteil - der mit einem Raum des zweiten Abschnitts in Verbindung steht, in dem das elastische Element angeordnet ist - kleiner ist als der Durchmesser des elastischen Elements, solange keine Zugkraft ausgeübt wird.

7. Tragbares Produkt nach Anspruch 6, wobei
die dehnbare Folie ferner einen dritten Abschnitt (B3) umfasst, der in der ersten Richtung zwischen dem ersten Abschnitt (B1) und dem zweiten Abschnitt (B2) angeordnet ist und in dem das elastische Element untergebracht ist, und
in dem dritten Abschnitt ein Bereich vorhanden ist, in dem in einem Querschnitt, der in einer zur ersten Richtung orthogonalen Richtung geschnitten ist, der maximale Abstand zwischen dem ersten Folienstück und dem zweiten Folienstück, der mit dem Raum des zweiten Abschnitts, in dem das elastische Element angeordnet ist, in Verbindung steht, kleiner ist als der Durchmesser des elastischen Elements, solange keine Spannung ausgeübt wird.

## Revendications

1. Dispositif de fabrication de feuilles extensibles (100) comprenant :
un dispositif de formage (200) configuré pour former un corps continu de feuille extensible (116) dans lequel sont intégrés une première feuille (114) qui est continue, une deuxième feuille (115) qui est continue, et un élément élastique (11) qui est continu et qui est disposé entre la première feuille et la deuxième feuille ; et
un dispositif de découpe (300) configuré pour découper l'élément élastique du corps continu de feuille extensible,
le dispositif de formage comportant un premier élément de jonction (210) configuré pour supporter la première feuille, l'élément élastique et la deuxième feuille, qui sont acheminés dans une direction d'acheminement (D), et un deuxième élément de jonction (230) configuré pour prendre en sandwich, conjointement avec le premier élément de jonction, la première feuille, l'élément élastique et la deuxième feuille,
le premier élément de jonction comportant une première saillie (212a) et une deuxième saillie (212b) disposée de manière adjacente à la première saillie dans la direction de transport, sur une surface (211) tournée vers le deuxième élément de jonction,
le deuxième élément d'assemblage est configuré pour :
former une partie de restriction (116b) dans le corps continu de feuille extensible en prenant en sandwich l'élément élastique, la première feuille et la deuxième feuille entre le deuxième élément d'assemblage et la première saillie du premier élément d'assemblage, puis en soudant ensemble la première feuille et la deuxième feuille ; et
former une partie fixe (116a) dans le corps continu de feuille extensible en prenant en sandwich l'élément élastique, la première feuille et la deuxième feuille entre le deuxième élément de jonction et la deuxième saillie du premier élément de jonction, puis en soudant au moins l'une parmi la première feuille et la deuxième feuille à l'élément élastique,
le dispositif de découpe est configuré pour découper l'élément élastique du corps continu de feuille extensible acheminé au niveau de la partie de restriction du corps continu de feuille extensible, et
dans la partie de restriction, la première feuille et la deuxième feuille n'étant pas soudées à l'élément élastique et le mouvement de contraction de l'élément élastique découpé par le dispositif de découpe étant limité par la première feuille et la deuxième feuille.

2. Dispositif de fabrication de feuilles extensibles selon la revendication 1, dans lequel la première saillie (212a) comprend une première partie de formation de rainure (214, 215) dans laquelle est formée une première rainure (214a, 215a) dans laquelle l'élément élastique et la deuxième feuille, acheminés sous tension dans la direction d'acheminement, sont conçus pour pénétrer, et qui s'étend le long de la direction d'acheminement,
la deuxième saillie (212b) comprend une deuxième partie de formation de rainure (216) dans laquelle est formée une deuxième rainure (216a) dans laquelle l'élément élastique et la deuxième feuille, acheminés dans la direction d'acheminement, sont destinés à s'engager et qui s'étend le long de la direction d'acheminement,
la profondeur de la première rainure est supérieure à une première valeur somme, qui correspond à la somme du diamètre de l'élément élastique et de l'épaisseur de la deuxième feuille acheminée sous tension à travers la première rainure dans la direction d'acheminement, et
la profondeur de la deuxième rainure est inférieure à la première valeur somme.

3. Dispositif de fabrication de feuilles extensibles selon la revendication 2, dans lequel
la profondeur de la première rainure (214a, 215a) est inférieure à une deuxième valeur de somme, qui correspond à la somme du diamètre de l'élément élastique et de l'épaisseur de la deuxième feuille lorsque aucune tension n'est appliquée.

4. Dispositif de fabrication de feuilles extensibles selon la revendication 2 ou 3, dans lequel
la largeur de la première rainure (214a, 215a) dans le sens de la largeur (S) du premier élément de jonction (210) est
supérieure à une troisième valeur somme, qui correspond à la somme du diamètre de l'élément élastique et de deux fois l'épaisseur de la deuxième feuille acheminée sous tension à travers la forme dans la direction de transport, et
inférieure à une quatrième valeur somme, qui correspond à la somme du diamètre de l'élément élastique et de deux fois l'épaisseur de la deuxième feuille lorsqu'aucune tension n'est appliquée.

5. Procédé de fabrication d'une feuille extensible à l'aide d'un dispositif de fabrication de feuilles extensibles (100) comprenant un dispositif de formage (200) et un dispositif de découpe (300), le procédé comprenant :
une étape de formage (P1) consistant à assembler, à l'aide du dispositif de formage, une première feuille (114) continue, une deuxième feuille (115) continue et un élément élastique (11) continu disposé entre la première feuille et la deuxième feuille, afin de former un corps continu de feuille extensible ; et
une étape de découpe (P2) consistant à découper, à l'aide du dispositif de découpe, l'élément élastique du corps continu de feuille extensible, dans laquelle
l'étape de formage comprend :
une étape de transport au cours de laquelle la première feuille, la deuxième feuille et l'élément élastique sont transportés dans une direction de transport (D) dans un état dans lequel l'élément élastique a été disposé entre la première feuille et la deuxième feuille ;
une première étape de formage au cours de laquelle l'élément élastique, la première feuille et la deuxième feuille, acheminés dans la direction de transport, sont pris en sandwich entre un premier élément de jonction (210) qui supporte la première feuille, l'élément élastique et la deuxième feuille, et un deuxième élément de jonction (230) afin de souder ensemble l'élément élastique et au moins l'une parmi la première feuille et la deuxième feuille, de manière à former une partie fixe (116a) dans le corps continu de feuille extensible ; et
une deuxième étape de formage dans laquelle l'élément élastique, la première feuille et la deuxième feuille sont pris en sandwich entre le premier élément de jonction et le deuxième élément de jonction afin de souder la première feuille et la deuxième feuille l'une à l'autre de manière à former une partie de restriction (116b) à un emplacement adjacent à la partie fixe sur le corps continu de feuille extensible,
lors de l'étape de découpe, l'élément élastique du corps continu en feuille extensible est découpé au niveau de la partie de restriction du corps continu en feuille extensible, et
au niveau de la partie de restriction, la première feuille et la deuxième feuille ne sont pas soudées à l'élément élastique, et le mouvement de contraction de l'élément élastique découpé lors de l'étape de découpe est limité par la première feuille et la deuxième feuille.

6. Article vestimentaire (1) comprenant une feuille extensible (10), dans lequel :
la feuille extensible comprend au moins une première partie de feuille (14), une deuxième partie de feuille (15) et un élément élastique (11) qui s'étend dans une première direction (D) et qui est disposé entre la première partie de feuille et la deuxième partie de feuille ;
la feuille extensible comprend, dans la première direction,
une première partie (B1) où l'élément élastique est absent ; et
une deuxième partie (B2) où l'élément élastique est soudé à au moins l'une des première et deuxième parties de feuille, et
dans la première partie, il existe une zone où, dans une vue en coupe transversale pratiquée dans une direction orthogonale à la première direction, l'écart maximal entre la première pièce de feuille et la deuxième pièce de feuille, qui communique avec un espace de la deuxième partie où est placé l'élément élastique, est inférieur au diamètre de l'élément élastique lorsqu'aucune tension n'est appliquée.

7. Article vestimentaire selon la revendication 6, dans lequel
la feuille extensible comprend en outre une troisième partie (B3) disposée entre la première partie (B1) et la deuxième partie (B2) dans la première direction et dans laquelle est placé l'élément élastique, et
dans la troisième partie, il existe une zone où, dans une vue en coupe transversale pratiquée dans une direction orthogonale à la première direction, l'écart maximal entre la première pièce de feuille et la deuxième pièce de feuille, qui communique avec l'espace de la deuxième partie où est placé l'élément élastique, est inférieur au diamètre de l'élément élastique lorsqu'aucune tension n'est appliquée.
